# EUROPEAN PATENT APPLICATION

(11) **EP 3 960 849 A1**
(43) Date of publication of application: **02.03.2022**
(21) Application number: 20795581.6
(22) Date of filing: 22.04.2020
(51) Int. Cl.: C12N 5/0783, A61K 35/17

(54) **ALLOGENEIC CAR-T CELLS, PREPARATION THEREOF AND USE THEREOF**

(30) Priority: 22.04.2019 CN 201910323948
(71) Applicant: Fundamenta Therapeutics Inc., Suzhou, Jiangsu 215200 (CN)
(72) Inventor: LI, Jun, Suzhou, Jiangsu 215200 (CN); ZHANG, Pengchao, Suzhou, Jiangsu 215200 (CN); XU, Zhao, Suzhou, Jiangsu 215200 (CN); HE, Ling, Suzhou, Jiangsu 215200 (CN); LIU, Weikang, Suzhou, Jiangsu 215200 (CN); JIANG, Yuchen, Suzhou, Jiangsu 215200 (CN); QIN, Hanxiao, Suzhou, Jiangsu 215200 (CN); XIA, Yu, Suzhou, Jiangsu 215200 (CN)
(74) Representative: EP&C
(86) International application number: PCT/CN2020/086032
(87) International publication number: WO 2020/216230

(57) **Abstract**

Provided are allogeneic CAR-T cells prepared by expressing related functional proteins in T cells and regulating HLA class I molecules on the cell surface. Also provided is the use of the allogeneic CAR-T cells.

Sequence Listing

<110>  FUNDAMENTA THERAPEUTICS INC.

<120>  ALLOGENEIC CAR-T CELL, PREPARATION THEREFOR, AND APPLICATION THEREOF

<130>  198073PCWO

<150>  CN201910323948.3
<151>  2019-04-22

<160>  12

<170>  SIPOSequenceListing 1.0

<210>  1
<211>  1848
<212>  DNA
<213>  Artificial Sequence

<220>
<223>  nucleic acid sequence of CD19-CAR-EHV1 UL49.5

<400>  1
atggatatga gggtgcctgc ccagctgctg ggcctgctgc tgctgtggct gaggggcgct      60
aggtgtgaca tccagatgac ccagagccct tcctccctga gcgcctccgt gggcgataga     120
gtgacaatca catgtagagc ctcccaggac atcagcaagt acctgaactg gtaccagcag     180
aagcccggca aggcccccaa gctgctgatc taccacacct ccagactgca cagcggcgtg     240
cctagcaggt tcagcggctc cggcagcggc accgacttta cactgaccat cagctccctg     300
cagcctgagg atttcgccac ctactactgt cagcagggca atacactgcc ctacaccttt     360
ggccagggca ccaagctgga gatcaaggga tccaccagcg gcggaggaag cggcggaggt     420
agcggaggag gcggaagctc ccaggtgaca ctgaaggaga gcggccctgc cctggtgaag     480
cctacacaga cactgacact gacgtgtacc ttctccggct tcagcctgtc cgattacggc     540
gtgagctgga tcagacagcc tcctggcaag gccctggagt ggctggccgt gatctggggc     600
agcgagacca cctactacaa ttccgccctg aagagcaggc tgaccatctc caaggacacc     660
tccaagaacc aggtggtgct gaccatgacc aatatggatc ctgtggacac cgccacatac     720
tactgtgcca agcactacta ctacggcggc agctacgcca tggattactg gggccagggc     780
accctggtga ccgtgagctc caccacgacg ccagcgccgc gaccaccaac accggcgccc     840
accatcgcgt cgcagcccct gtccctgcgc ccagaggcgt gccggccagc ggcggggggc     900
gcagtgcaca cgagggggct ggacttcgcc tgtgatatct acatctgggc gcccttggcc     960
gggacttgtg gggtccttct cctgtcactg gttatcaccc tttactgcaa acggggcaga    1020
aagaaactcc tgtatatatt caaacaacca tttatgagac cagtacaaac tactcaagag    1080
gaagatggct gtagctgccg atttccagaa gaagaagaag gaggatgtga actgagagtg    1140
aagttcagca ggagcgcaga cgcccccgcg taccagcagg gccagaacca gctctataac    1200
gagctcaatc taggacgaag agaggagtac gatgttttgg acaagagacg tggccgggac    1260
cctgagatgg ggggaaagcc gagaaggaag aaccctcagg aaggcctgta caatgaactg    1320
cagaaagata agatggcgga ggcctacagt gagattggga tgaaaggcga gcgccggagg    1380
ggcaaggggc acgatggcct ttaccagggt ctcagtacag ccaccaagga cacctacgac    1440
gcccttcaca tgcaggccct gccccctcgc ggctccggag taaagcaaac actgaacttt    1500
gaccttctca agttggctgg agacgttgag tccaatcctg ggcccatgct gtccacgaga    1560
ttcgtgacgc tggccattct cgcctgcctt ttggtggtgc ttggtctggc cagaggggct    1620
ggtggcgacc caggtgtgaa gcaacgaatc gacgttgcta gagaagagga gagacgcgac    1680
ttctggcatg cagcctgctc cggacacgga ttcccaatta ccaccccaag cacggctgct    1740
attctatttt atgtgtctct gcttgcagtg ggagtggctg ttgcctgcca ggcataccgc    1800
gccgtcttgc gaatcgtgac gctggagatg ttgcaacacc tgcattga                 1848

<210>  2
<211>  615
<212>  PRT
<213>  Artificial Sequence

<220>
<223>  amino acid sequence of CD19-CAR-EHV1 UL49.5

<400> 2
Met Asp Met Arg Val Pro Ala Gln Leu Leu Gly Leu Leu Leu Leu Trp
1               5                   10                  15
Leu Arg Gly Ala Arg Cys Asp Ile Gln Met Thr Gln Ser Pro Ser Ser
            20                  25                  30
Leu Ser Ala Ser Val Gly Asp Arg Val Thr Ile Thr Cys Arg Ala Ser
        35                  40                  45
Gln Asp Ile Ser Lys Tyr Leu Asn Trp Tyr Gln Gln Lys Pro Gly Lys
    50                  55                  60
Ala Pro Lys Leu Leu Ile Tyr His Thr Ser Arg Leu His Ser Gly Val
65                  70                  75                  80
Pro Ser Arg Phe Ser Gly Ser Gly Ser Gly Thr Asp Phe Thr Leu Thr
                85                  90                  95
Ile Ser Ser Leu Gln Pro Glu Asp Phe Ala Thr Tyr Tyr Cys Gln Gln
            100                 105                 110
Gly Asn Thr Leu Pro Tyr Thr Phe Gly Gln Gly Thr Lys Leu Glu Ile
        115                 120                 125
Lys Gly Ser Thr Ser Gly Gly Gly Ser Gly Gly Gly Ser Gly Gly Gly
    130                 135                 140
Gly Ser Ser Gln Val Thr Leu Lys Glu Ser Gly Pro Ala Leu Val Lys
145                 150                 155                 160
Pro Thr Gln Thr Leu Thr Leu Thr Cys Thr Phe Ser Gly Phe Ser Leu
                165                 170                 175
Ser Asp Tyr Gly Val Ser Trp Ile Arg Gln Pro Pro Gly Lys Ala Leu
            180                 185                 190
Glu Trp Leu Ala Val Ile Trp Gly Ser Glu Thr Thr Tyr Tyr Asn Ser
        195                 200                 205
Ala Leu Lys Ser Arg Leu Thr Ile Ser Lys Asp Thr Ser Lys Asn Gln
    210                 215                 220
Val Val Leu Thr Met Thr Asn Met Asp Pro Val Asp Thr Ala Thr Tyr
225                 230                 235                 240
Tyr Cys Ala Lys His Tyr Tyr Tyr Gly Gly Ser Tyr Ala Met Asp Tyr
                245                 250                 255
Trp Gly Gln Gly Thr Leu Val Thr Val Ser Ser Thr Thr Thr Pro Ala
            260                 265                 270
Pro Arg Pro Pro Thr Pro Ala Pro Thr Ile Ala Ser Gln Pro Leu Ser
        275                 280                 285
Leu Arg Pro Glu Ala Cys Arg Pro Ala Ala Gly Gly Ala Val His Thr
    290                 295                 300
Arg Gly Leu Asp Phe Ala Cys Asp Ile Tyr Ile Trp Ala Pro Leu Ala
305                 310                 315                 320
Gly Thr Cys Gly Val Leu Leu Leu Ser Leu Val Ile Thr Leu Tyr Cys
                325                 330                 335
Lys Arg Gly Arg Lys Lys Leu Leu Tyr Ile Phe Lys Gln Pro Phe Met
            340                 345                 350
Arg Pro Val Gln Thr Thr Gln Glu Glu Asp Gly Cys Ser Cys Arg Phe
        355                 360                 365
Pro Glu Glu Glu Glu Gly Gly Cys Glu Leu Arg Val Lys Phe Ser Arg
    370                 375                 380
Ser Ala Asp Ala Pro Ala Tyr Gln Gln Gly Gln Asn Gln Leu Tyr Asn
385                 390                 395                 400
Glu Leu Asn Leu Gly Arg Arg Glu Glu Tyr Asp Val Leu Asp Lys Arg
                405                 410                 415
Arg Gly Arg Asp Pro Glu Met Gly Gly Lys Pro Arg Arg Lys Asn Pro
            420                 425                 430
Gln Glu Gly Leu Tyr Asn Glu Leu Gln Lys Asp Lys Met Ala Glu Ala
        435                 440                 445
Tyr Ser Glu Ile Gly Met Lys Gly Glu Arg Arg Arg Gly Lys Gly His
    450                 455                 460
Asp Gly Leu Tyr Gln Gly Leu Ser Thr Ala Thr Lys Asp Thr Tyr Asp
465                 470                 475                 480
Ala Leu His Met Gln Ala Leu Pro Pro Arg Gly Ser Gly Val Lys Gln

485                 490                 495
Thr Leu Asn Phe Asp Leu Leu Lys Leu Ala Gly Asp Val Glu Ser Asn
            500                 505                 510
Pro Gly Pro Met Leu Ser Thr Arg Phe Val Thr Leu Ala Ile Leu Ala
        515                 520                 525
Cys Leu Leu Val Val Leu Gly Leu Ala Arg Gly Ala Gly Gly Asp Pro
    530                 535                 540
Gly Val Lys Gln Arg Ile Asp Val Ala Arg Glu Glu Glu Arg Arg Asp
545                 550                 555                 560
Phe Trp His Ala Ala Cys Ser Gly His Gly Phe Pro Ile Thr Thr Pro
                565                 570                 575
Ser Thr Ala Ala Ile Leu Phe Tyr Val Ser Leu Leu Ala Val Gly Val
            580                 585                 590
Ala Val Ala Cys Gln Ala Tyr Arg Ala Val Leu Arg Ile Val Thr Leu
        595                 600                 605
Glu Met Leu Gln His Leu His
    610                 615

<210>  3
<211>  21
<212>  DNA
<213>  Artificial Sequence

<220>
<223>  Primer

<400>  3
tcaagcctca gacagtggtt c                                                21

<210>  4
<211>  2193
<212>  DNA
<213>  Artificial Sequence

<220>
<223>  nucleic acid sequence of CD19-CAR-US11

<400>  4
atggatatga gggtgcctgc ccagctgctg ggcctgctgc tgctgtggct gaggggcgct      60
aggtgtgaca tccagatgac ccagagccct tcctccctga gcgcctccgt gggcgataga     120
gtgacaatca catgtagagc ctcccaggac atcagcaagt acctgaactg gtaccagcag     180
aagcccggca aggcccccaa gctgctgatc taccacacct ccagactgca cagcggcgtg     240
cctagcaggt tcagcggctc cggcagcggc accgacttta cactgaccat cagctccctg     300
cagcctgagg atttcgccac ctactactgt cagcagggca atacactgcc ctacaccttt     360
ggccagggca ccaagctgga gatcaaggga tccaccagcg gcggaggaag cggcggaggt     420
agcggaggag gcggaagctc ccaggtgaca ctgaaggaga gcggccctgc cctggtgaag     480
cctacacaga cactgacact gacgtgtacc ttctccggct tcagcctgtc cgattacggc     540
gtgagctgga tcagacagcc tcctggcaag gccctggagt ggctggccgt gatctggggc     600
agcgagacca cctactacaa ttccgccctg aagagcaggc tgaccatctc caaggacacc     660
tccaagaacc aggtggtgct gaccatgacc aatatggatc ctgtggacac cgccacatac     720
tactgtgcca agcactacta ctacggcggc agctacgcca tggattactg gggccagggc     780
accctggtga ccgtgagctc caccacgacg ccagcgccgc gaccaccaac accggcgccc     840
accatcgcgt cgcagcccct gtccctgcgc ccagaggcgt gccggccagc ggcggggggc     900
gcagtgcaca cgagggggct ggacttcgcc tgtgatatct acatctgggc gcccttggcc     960
gggacttgtg gggtccttct cctgtcactg gttatcaccc tttactgcaa acggggcaga    1020
aagaaactcc tgtatatatt caaacaacca tttatgagac cagtacaaac tactcaagag    1080
gaagatggct gtagctgccg atttccagaa gaagaagaag gaggatgtga actgagagtg    1140
aagttcagca ggagcgcaga cgcccccgcg taccagcagg gccagaacca gctctataac    1200
gagctcaatc taggacgaag agaggagtac gatgttttgg acaagagacg tggccgggac    1260
cctgagatgg ggggaaagcc gagaaggaag aaccctcagg aaggcctgta caatgaactg    1320
cagaaagata agatggcgga ggcctacagt gagattggga tgaaaggcga gcgccggagg    1380
ggcaaggggc acgatggcct ttaccagggt ctcagtacag ccaccaagga cacctacgac    1440
gcccttcaca tgcaggccct gccccctcgc ggctccggag taaagcaaac actgaacttt    1500

gaccttctca agttggctgg agacgttgag tccaatcctg ggcccatgaa ccttgtaatg 1560
cttattctag ccctctgggc cccggtcgcg ggtagtatgc ctgaattatc cttgactctt 1620
ttcgatgaac ctccgccctt ggtggagacg gagccgttac cgcctctgtc cgatgtttcg 1680
gagtaccgag tagagtattc cgaggcgcgc tgcgtgctcc gatcgggcgg tcgactggag 1740
gctctgtgga ccctgcgcgg gaacctgtcc gtgcccacgc cgacaccccg ggtgtactac 1800
cagacgctgg agggctacgc ggatcgagtg ccgacgccgg tggaggacgt ctccgaaagc 1860
ctcgtcgcaa aacgctactg gctccgggac tatcgtgttc cccaacgcac aaaactcgtg 1920
ttgttctact tttccccctg ccaccaatgc caaacttatt atgtagagtg cgaaccccgg 1980
tgcctcgtgc cttgggttcc cctgtggagc tcgttagagg acatcgaacg actattgttc 2040
gaagatcgcc gtctaatggc gtactacgcg ctcacgatta agtcggcgca gtatacgctg 2100
atgatggtgg cagtgattca agtgttttgg gggctgtatg tgaaaggttg gctgcaccga 2160
cattttccct ggatgttttc ggaccagtgg tga 2193

<210> 5
<211> 2097
<212> DNA
<213> Artificial Sequence

<220>
<223> nucleic acid sequence of CD19-CAR-US6

<400> 5
atggatatga gggtgcctgc ccagctgctg ggcctgctgc tgctgtggct gaggggcgct 60
aggtgtgaca tccagatgac ccagagccct tcctccctga gcgcctccgt gggcgataga 120
gtgacaatca catgtagagc ctcccaggac atcagcaagt acctgaactg gtaccagcag 180
aagcccggca aggcccccaa gctgctgatc taccacacct ccagactgca cagcggcgtg 240
cctagcaggt tcagcggctc cggcagcggc accgacttta cactgaccat cagctccctg 300
cagcctgagg atttcgccac ctactactgt cagcagggca atacactgcc ctacaccttt 360
ggccagggca ccaagctgga gatcaaggga tccaccagcg gcggaggaag cggcggaggt 420
agcggaggag gcggaagctc ccaggtgaca ctgaaggaga gcggccctgc cctggtgaag 480
cctacacaga cactgacact gacgtgtacc ttctccggct tcagcctgtc cgattacggc 540
gtgagctgga tcagacagcc tcctggcaag gccctggagt ggctggccgt gatctggggc 600
agcgagacca cctactacaa ttccgccctg aagagcaggc tgaccatctc caaggacacc 660
tccaagaacc aggtggtgct gaccatgacc aatatggatc ctgtggacac cgccacatac 720
tactgtgcca agcactacta ctacggcggc agctacgcca tggattactg gggccagggc 780
accctggtga ccgtgagctc caccacgacg ccagcgccgc gaccaccaac accggcgccc 840
accatcgcgt cgcagcccct gtccctgcgc ccagaggcgt gccggccagc ggcggggggc 900
gcagtgcaca cgagggggct ggacttcgcc tgtgatatct acatctgggc gcccttggcc 960
gggacttgtg gggtccttct cctgtcactg gttatcaccc tttactgcaa acggggcaga 1020
aagaaactcc tgtatatatt caaacaacca tttatgagac cagtacaaac tactcaagag 1080
gaagatggct gtagctgccg atttccagaa gaagaagaag gaggatgtga actgagagtg 1140
aagttcagca ggagcgcaga cgcccccgcg taccagcagg gccagaacca gctctataac 1200
gagctcaatc taggacgaag agaggagtac gatgttttgg acaagagacg tggccgggac 1260
cctgagatgg ggggaaagcc gagaaggaag aaccctcagg aaggcctgta caatgaactg 1320
cagaaagata agatggcgga ggcctacagt gagattggga tgaaaggcga gcgccggagg 1380
ggcaaggggc acgatggcct ttaccagggt ctcagtacag ccaccaagga cacctacgac 1440
gcccttcaca tgcaggccct gccccctcgc ggctccggag taaagcaaac actgaacttt 1500
gaccttctca agttggctgg agacgttgag tccaatcctg ggcccatgga tctcttgatt 1560
cgtctcggtt ttctgttgat gtgtgcgttg ccgacccccg gtgagcggtc ttcgcgtgac 1620
ccgaaaaccc ttctctctct gtctccgcga caacaagctt gtgttccgag aacgaagtcg 1680
cacagacccg tttgttacaa cgatacaggg gactgcacag atgcagatga tagctggaaa 1740
cagctgggtg aggactttgc gcaccaatgc ttgcaggcgg cgaaaaagag gcctaaaacg 1800
cacaaatccc gtccgaacga taggaacctt gagggtaggc tgacctgtca acgagtccgt 1860
cggctactgc cctgtgattt ggatattcat cctagccacc ggttgttaac gcttatgaat 1920
aactgcgtct gtgacggggc cgtttggaac gcgtttcgct tgatagaacg acacggattc 1980
ttcgctgtga ctttgtattt atgttgcggg attaccctgc tggttgttat tctagcattg 2040
ctgtgcagca taacatacga atcgactgga cgtgggattc gacgttgtgg ctcctga 2097

<210> 6
<211> 1836
<212> DNA
<213> Artificial Sequence

<220>
<223>  nucleic acid sequence of CD19-CAR-BHV1 UL49.5

<400>  6
atggatatga gggtgcctgc ccagctgctg ggcctgctgc tgctgtggct gaggggcgct      60
aggtgtgaca tccagatgac ccagagccct tcctccctga gcgcctccgt gggcgataga     120
gtgacaatca catgtagagc ctcccaggac atcagcaagt acctgaactg gtaccagcag     180
aagcccggca aggcccccaa gctgctgatc taccacacct ccagactgca cagcggcgtg     240
cctagcaggt tcagcggctc cggcagcggc accgacttta cactgaccat cagctccctg     300
cagcctgagg atttcgccac ctactactgt cagcagggca atacactgcc ctacaccttt     360
ggccagggca ccaagctgga gatcaaggga tccaccagcg gcggaggaag cggcggaggt     420
agcggaggag gcggaagctc ccaggtgaca ctgaaggaga gcggccctgc cctggtgaag     480
cctacacaga cactgacact gacgtgtacc ttctccggct tcagcctgtc cgattacggc     540
gtgagctgga tcagacagcc tcctggcaag gccctggagt ggctggccgt gatctggggc     600
agcgagacca cctactacaa ttccgccctg aagagcaggc tgaccatctc caaggacacc     660
tccaagaacc aggtggtgct gaccatgacc aatatggatc ctgtggacac cgccacatac     720
tactgtgcca agcactacta ctacggcggc agctacgcca tggattactg gggccagggc     780
accctggtga ccgtgagctc caccacgacg ccagcgccgc gaccaccaac accggcgccc     840
accatcgcgt cgcagcccct gtccctgcgc ccagaggcgt gccggccagc ggcggggggc     900
gcagtgcaca cgagggggct ggacttcgcc tgtgatatct acatctgggc gcccttggcc     960
gggacttgtg gggtccttct cctgtcactg gttatcaccc tttactgcaa acggggcaga    1020
aagaaactcc tgtatatatt caaacaacca tttatgagac cagtacaaac tactcaagag    1080
gaagatggct gtagctgccg atttccagaa gaagaagaag gaggatgtga actgagagtg    1140
aagttcagca ggagcgcaga cgcccccgcg taccagcagg gccagaacca gctctataac    1200
gagctcaatc taggacgaag agaggagtac gatgttttgg acaagagacg tggccgggac    1260
cctgagatgg ggggaaagcc gagaaggaag aaccctcagg aaggcctgta caatgaactg    1320
cagaaagata agatggcgga ggcctacagt gagattggga tgaaaggcga gcgccggagg    1380
ggcaaggggc acgatggcct ttaccagggt ctcagtacag ccaccaagga cacctacgac    1440
gcccttcaca tgcaggccct gccccctcgc ggctccggag taaagcaaac actgaacttt    1500
gaccttctca agttggctgg agacgttgag tccaatcctg ggcccatgcc gcggtcgccg    1560
ctcatcgttg cggttgtggc cgccgcgctg tttgccatcg tgcgcggccg cgaccccctg    1620
ctagacgcga tgcggcgcga gggggcaatg gacttttgga gcgcaggctg ctacgcgcgc    1680
ggggtgccgc tctcggagcc accgcaggcc ctggttgttt tttacgtggc cctgaccgcg    1740
gtaatggtcg ccgtggccct gtacgcgtac gggctttgct ttaggctcat gggcgccagc    1800
gggcccaata aaaaggagtc gcgggggcgg ggctga                              1836

<210>  7
<211>  2316
<212>  DNA
<213>  Artificial Sequence

<220>
<223>  nucleic acid sequence of CD19-CAR-k5

<400>  7
atggatatga gggtgcctgc ccagctgctg ggcctgctgc tgctgtggct gaggggcgct      60
aggtgtgaca tccagatgac ccagagccct tcctccctga gcgcctccgt gggcgataga     120
gtgacaatca catgtagagc ctcccaggac atcagcaagt acctgaactg gtaccagcag     180
aagcccggca aggcccccaa gctgctgatc taccacacct ccagactgca cagcggcgtg     240
cctagcaggt tcagcggctc cggcagcggc accgacttta cactgaccat cagctccctg     300
cagcctgagg atttcgccac ctactactgt cagcagggca atacactgcc ctacaccttt     360
ggccagggca ccaagctgga gatcaaggga tccaccagcg gcggaggaag cggcggaggt     420
agcggaggag gcggaagctc ccaggtgaca ctgaaggaga gcggccctgc cctggtgaag     480
cctacacaga cactgacact gacgtgtacc ttctccggct tcagcctgtc cgattacggc     540
gtgagctgga tcagacagcc tcctggcaag gccctggagt ggctggccgt gatctggggc     600
agcgagacca cctactacaa ttccgccctg aagagcaggc tgaccatctc caaggacacc     660
tccaagaacc aggtggtgct gaccatgacc aatatggatc ctgtggacac cgccacatac     720
tactgtgcca agcactacta ctacggcggc agctacgcca tggattactg gggccagggc     780
accctggtga ccgtgagctc caccacgacg ccagcgccgc gaccaccaac accggcgccc     840
accatcgcgt cgcagcccct gtccctgcgc ccagaggcgt gccggccagc ggcggggggc     900
gcagtgcaca cgagggggct ggacttcgcc tgtgatatct acatctgggc gcccttggcc     960
gggacttgtg gggtccttct cctgtcactg gttatcaccc tttactgcaa acggggcaga    1020
aagaaactcc tgtatatatt caaacaacca tttatgagac cagtacaaac tactcaagag    1080

gaagatggct gtagctgccg atttccagaa gaagaagaag gaggatgtga actgagagtg 1140
aagttcagca ggagcgcaga cgcccccgcg taccagcagg gccagaacca gctctataac 1200
gagctcaatc taggacgaag agaggagtac gatgttttgg acaagagacg tggccgggac 1260
cctgagatgg ggggaaagcc gagaaggaag aaccctcagg aaggcctgta caatgaactg 1320
cagaaagata agatggcgga ggcctacagt gagattggga tgaaaggcga gcgccggagg 1380
ggcaaggggc acgatggcct ttaccagggt ctcagtacag ccaccaagga cacctacgac 1440
gcccttcaca tgcaggccct gccccctcgc ggctccggag taaagcaaac actgaacttt 1500
gaccttctca agttggctgg agacgttgag tccaatcctg ggcccatggc gtccaaggac 1560
gtagaagagg gtgtagaggg acccatctgc tggatatgta gggaagaggt ggggaacgag 1620
ggcatacacc cctgcgcctg taccggagag ctggatgtcg tccacccgca gtgtttaagc 1680
acttggctaa cagtgtctcg aaacacggcc tgtcaaatgt gtcgcgttat ataccgcacg 1740
cgcacgcagt ggcgtagtcg ccttaacctg tggccggaga tggagcgcca agaaattttt 1800
gaactgtttt tgctgatgtc tgtggtggtg gctgggcttg tcggcgtggc gttgtgcacc 1860
tggacgctcc tggtcatcct aactgctcct gcgggaacat tctcccccgg ggccgtgctc 1920
ggttttctct gcttctttgg gttttaccaa atatttattg tgtttgcatt tggcggcata 1980
tgccgcgtaa gtggcactgt gagggcatta tacgcggcaa ataacacccg ggtgaccgta 2040
ctgccatacc gacggccgcg ccggccaacc gcgaacgaag ataacatcga attgacggtc 2100
cttgtcggac ccgcgggcgg gacggacgag gagcccacgg acgagtcatc tgaaggagac 2160
gtcgcctctg gagacaaaga acgtgacggt tcatccggag acgagccgga cgggggcccg 2220
aacgaccgtg cgggacttag ggggacagcg cgtaccgacc tatgcgcgcc cacaaaaaag 2280
ccggtgcgga aaaatcatcc aaaaaacaac ggttga 2316

<210> 8
<211> 291
<212> DNA
<213> Artificial Sequence

<220>
<223> BHV-1 UL49.5

<400> 8
atgccgcggt cgccgctcat cgttgcggtt gtggccgccg cgctgtttgc catcgtgcgc 60
ggccgcgacc ccctgctaga cgcgatgcgg cgcgaggggg caatggactt ttggagcgca 120
ggctgctacg cgcgcggggt gccgctctcg gagccaccgc aggccctggt tgttttttac 180
gtggccctga ccgcggtaat ggtcgccgtg gccctgtacg cgtacgggct ttgctttagg 240
ctcatgggcg ccagcgggcc caataaaaag gagtcgcggg ggcggggctg a 291

<210> 9
<211> 303
<212> DNA
<213> Artificial Sequence

<220>
<223> EHV-1 UL49.5

<400> 9
atgctgtcca cgagattcgt gacgctggcc attctcgcct gccttttggt ggtgcttggt 60
ctggccagag gggctggtgg cgacccaggt gtgaagcaac gaatcgacgt tgctagagaa 120
gaggagagac gcgacttctg gcatgcagcc tgctccggac acggattccc aattaccacc 180
ccaagcacgg ctgctattct attttatgtg tctctgcttg cagtgggagt ggctgttgcc 240
tgccaggcat accgcgccgt cttgcgaatc gtgacgctgg agatgttgca acacctgcat 300
tga 303

<210> 10
<211> 552
<212> DNA
<213> Artificial Sequence

<220>
<223> HCMV US6

<400> 10
atggatctct tgattcgtct cggttttctg ttgatgtgtg cgttgccgac ccccggtgag 60

cggtcttcgc gtgacccgaa aacccttctc tctctgtctc cgcgacaaca agcttgtgtt 120
ccgagaacga agtcgcacag acccgtttgt tacaacgata caggggactg cacagatgca 180
gatgatagct ggaaacagct gggtgaggac tttgcgcacc aatgcttgca ggcggcgaaa 240
aagaggccta aaacgcacaa atcccgtccg aacgatagga accttgaggg taggctgacc 300
tgtcaacgag tccgtcggct actgccctgt gatttggata ttcatcctag ccaccggttg 360
ttaacgctta tgaataactg cgtctgtgac ggggccgttt ggaacgcgtt tcgcttgata 420
gaacgacacg gattcttcgc tgtgactttg tatttatgtt gcgggattac cctgctggtt 480
gttattctag cattgctgtg cagcataaca tacgaatcga ctggacgtgg gattcgacgt 540
tgtggctcct ga 552

<210> 11
<211> 648
<212> DNA
<213> Artificial Sequence

<220>
<223> HCMV US11

<400> 11
atgaaccttg taatgcttat tctagccctc tgggccccgg tcgcgggtag tatgcctgaa 60
ttatccttga ctcttttcga tgaacctccg cccttggtgg agacggagcc gttaccgcct 120
ctgtccgatg tttcggagta ccgagtagag tattccgagg cgcgctgcgt gctccgatcg 180
ggcggtcgac tggaggctct gtggaccctg cgcgggaacc tgtccgtgcc cacgccgaca 240
ccccgggtgt actaccagac gctggagggc tacgcggatc gagtgccgac gccggtggag 300
gacgtctccg aaagcctcgt cgcaaaacgc tactggctcc gggactatcg tgttccccaa 360
cgcacaaaac tcgtgttgtt ctacttttcc ccctgccacc aatgccaaac ttattatgta 420
gagtgcgaac cccggtgcct cgtgccttgg gttcccctgt ggagctcgtt agaggacatc 480
gaacgactat tgttcgaaga tcgccgtcta atggcgtact acgcgctcac gattaagtcg 540
gcgcagtata cgctgatgat ggtggcagtg attcaagtgt tttgggggct gtatgtgaaa 600
ggttggctgc accgacattt tccctggatg ttttcggacc agtggtga 648

<210> 12
<211> 771
<212> DNA
<213> Artificial Sequence

<220>
<223> KSHV k5

<400> 12
atggcgtcca aggacgtaga agagggtgta gagggaccca tctgctggat atgtagggaa 60
gaggtgggga acgagggcat acacccctgc gcctgtaccg gagagctgga tgtcgtccac 120
ccgcagtgtt taagcacttg gctaacagtg tctcgaaaca cggcctgtca aatgtgtcgc 180
gttatatacc gcacgcgcac gcagtggcgt agtcgcctta acctgtggcc ggagatggag 240
cgccaagaaa tttttgaact gtttttgctg atgtctgtgg tggtggctgg gcttgtcggc 300
gtggcgttgt gcacctggac gctcctggtc atcctaactg ctcctgcggg aacattctcc 360
cccggggccg tgctcggttt tctctgcttc tttgggtttt accaaatatt tattgtgttt 420
gcatttggcg gcatatgccg cgtaagtggc actgtgaggg cattatacgc ggcaaataac 480
acccgggtga ccgtactgcc ataccgacgg ccgcgccggc caaccgcgaa cgaagataac 540
atcgaattga cggtccttgt cggacccgcg ggcgggacgg acgaggagcc cacggacgag 600
tcatctgaag gagacgtcgc ctctggagac aaagaacgtg acggttcatc cggagacgag 660
ccggacgggg gcccgaacga ccgtgcggga cttaggggga cagcgcgtac cgacctatgc 720
gcgcccacaa aaaagccggt gcggaaaaat catccaaaaa acaacggttg a 771

## Description

### Technical field

The disclosure relates to specific allogeneic CAR-T cells, their preparation and use.

### Background

With the development of tumor therapy, chimeric antigen receptor-T (CAR-T) immunotherapy has gradually become a treatment of great concern. A CAR expressed by CAR-T cells generally comprises extracellular antigen binding domain, transmembrane domain and intracellular signaling domain. CAR-T cells could effectively recognize tumor antigens and produce specific antitumor immune response, which is not limited by major histocompatibility complex (MHC). At present, the US FDA has approved the listing of two autologous CAR-T cell products, namely Kymriah of Novartis and YesCAR-Ta of Kate, for the treatment of refractory recurrent non-Hodgkin lymphoma and acute B-lymphocytic leukemia. A large number of clinical trials have proved that CAR-T has great antitumor potential as a personalized living cell drug (Maude et al. 2018; Park et al. 2018; Schuster et al. 2017).

The two CAR-T cell products on the market are obtained by collecting the patient's own peripheral blood, isolating T cells, transfecting the CAR gene expression frame into T cells with lentivirus or retroviral vector, expanding culture, and then reinfusing into the patient. CAR-T of each patient needs to be prepared separately, the production period is long, and a variety of uncertain factors may lead to premature death or failure of CAR treatment, such as insufficient number of isolated cells, quality or dysfunction of T cells from the patient, failure of CAR-T cell preparation, rapid progress of patient diseases in the process of cell preparation, etc.

Batch preparation of ready to use allogeneic CAR-T cells from healthy donor T cells can effectively solve the problems in the preparation and use of autologous CAR-T cells. A successful allogeneic CAR-T cell technology needs to solve two key problems: (1) the TCR (T cell receptor) on the T cell surface of healthy donors will recognize the allogeneic antigen of patients, resulting in dangerous or even fatal graft-versus-host disease (GVHD); and (2) the host's immune system will recognize HLA (human leucocyte antigen) class I molecules on the surface of healthy donor T cells, resulting in the rapid clearance of reinfusion allogeneic T cells and affecting the antitumor efficacy of allogeneic CAR-T.

At present, it is consensus in the art that allogeneic T cells with TCR and HLA class I molecules removed can be effectively used in allogeneic CAR-T cell therapy. Because gene editing technology can efficiently and completely eliminate the expression of one or more targeted genes at the genomic level, it has quickly become the mainstream technical means for preparing allogeneic CAR-T to completely knock out the expression of TCR and HLA class I molecules of allogeneic CAR-T cells, which is expected to effectively prevent GVHD and host rejection in clinic. At present, a variety of gene editing technologies (zinc-finger nucleases, ZFN; transcription activator-like effector nucleases, TALENs; clustered regularly interspaced short palindromic repeats, CRISPR; ARCUS Genome Editing) have been actively applied to the preclinical development and clinical trials of allogeneic CAR-T cells (Provasi et al. 2012; Berdien et al. 2014; Ren et al. 2017; McCreedy et al. 2018).

### Summary

The first aspect of the disclosure provides an engineered T cell, wherein the engineered T cell expresses a functional protein capable of regulating the expression of HLA class I molecules on the cell surface, wherein the expression of HLA-A and HLA-B on the cell surface of the engineered T cell and the expression of HLA-E are differentially regulated, or the expression of HLA-A, HLA-B and HLA-E on the cell surface of the engineered T cell are down regulated.

In one or more embodiments, differential regulation is that the expression of HLA-A and HLA-B on the cell surface of the engineered T cells is down regulated, but the expression of HLA-E is not down regulated.

In one or more embodiments, the expression level of HLA class I molecules on the cell surface of the engineered T cells is 50% or less, preferably 10-50% of the T cells that do not express the functional protein.

In one or more embodiments, the expression level of HLA-A and/or HLA-B molecules on the surface of the engineered T cells is 5-50%, 10-50%, 10-45%, 10-40%, 15-50%, 20-50%, or 20-45% of the T cells that do not express the functional protein.

In one or more embodiments, the expression level of HLA-E molecules on the surface of the engineered T cells is 65-100%, 70-100%, 75-100%, 80-100%, 85-100%, 90-100%, 95-100% of the T cells that do not express the functional protein.

In one or more embodiments, the engineered T cell further expresses the chimeric antigen receptor, so that the engineered T cell is a CAR-T cell, wherein the CAR-T cell contains the coding sequence of the chimeric antigen receptor and that of the functional protein.

In one or more embodiments, the CAR-T cell contains the expression frame of the chimeric antigen receptor and that of the functional protein, or the coding sequence of the chimeric antigen receptor and that of the functional protein are in the same expression frame.

In one or more embodiments, the chimeric antigen receptor specifically binds one or more tumor antigens selected from the group consisting of EGFRvIII, mesothelin, gD2, Tn antigen, sTn antigen, Tn-O-glycopeptide, sTn-O-glycopeptide, PSMA, CD97, TAG72, CD44v6, CEA, EpCAM, KIT, IL-13RA2, Leguman, GD3, CD171, IL-11RA, PSCA, MAD-CT-1, MAD-CT-2, VEGFR2, LewisY, CD24, PDGFR-β, SSEA-4, folate receptor α, ERBB, HER2/neu, MUC1, EGFR, NCAM, Ephrin B2, CAIX, LMP2, SLE, HMWMAA, o-acetyl-GD2, folate receptor β, TEM1/CD248, TEM7R, FAP, Legumain, HPV E6 or E7, ML-IAP, CLDN6, TSHR, GPRC5d, ALK, polysialic acid, FOS related antigen, neutrophil elastase, TRP-2, CYP1B1, sperm protein 17, β Human chorionic gonadotropin, AFP, thyroglobulin, PLAC1, globoH, RAGE1, MN-CA IX, human telomerase reverse transcriptase, intestinal carboxylesterase, mut HSP 70-2, NA-17, NY-BR-1, UPK2, HAVCR1, ADRB3, PANX3, NY-ESO-1, GPR20, Ly6k, OR51E2, TARP, GFRα4 and the polypeptide fragments of any of these antigens presented on MHC, as well as CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79A, CD79B, CD80, CD81, CD82, CD83, CD84, CD85, CD86, CD123, CD135, CD138, CD179, CD269, Flt3, ROR1, BCMA, FcRn5, FcRn2, CS-1, CXCR4, CXCR5, CXCR7, IL-7/3R, IL-7/4/3R, and IL4R.

In one or more embodiments, the functional protein capable of regulating the expression of HLA class I molecules on the cell surface is selected from the group consisting of HSV, BHV-1, EHV-1/4, PRV, HSV-1/2, VZV, EBV, hCMV, mCMV, RhCMV, HHV-6/7, KSHV, MHV-68, the functional protein capable of directly target-degrading HLA class I molecules in cowpox virus and adenovirus, the functional protein capable of directly target-degrading HLA class I molecules through TAP protein, and the functional protein capable of down regulating the expression of HLA class I molecules by lysosomes.

In one or more embodiments, the functional protein is selected from: proteins US11 and US6 from HCMV, protein UL49.5 from BHV-1, protein UL49.5 from EHV-1 and protein k5 from KSHV.

In one or more embodiments, the functional protein is selected from: protein US 11 from HCMV and protein k5 from KSHV, and the expression HLA-A and HLA-B and the expression of HLA-E on the cell surface of the engineered T cells are differentially regulated. Preferably, the cell surface HLA-A and HLA-B expression of the engineered T cells are down regulated, but the HLA-E expression is not down regulated.

In one or more embodiments, the functional protein is selected from: protein US6 from HCMV, protein UL49.5 from BHV-1 and protein UL49.5 from EHV-1. The genotype of HLA-E of the engineered T cell is 01:03/01:03, and the expression of HLA-A and HLA-B and the expression of HLA-E on the cell surface of the engineered T cell are differentially regulated. Preferably, on the cell surface of the engineered T cell is down regulated, the expression of HLA-A and HLA-B, but the expression of HLA-E is not down regulated. The functional protein is preferably protein UL49.5 from EHV-1.

In one or more embodiments, the functional protein is selected from: protein US6 from HCMV, protein UL49.5 from BHV-1 and protein UL49.5 from EHV-1. The genotype of HLA-E of the engineered T cell is 01:01/01:01, and the expression of HLA-A, HLA-B and HLA-E on the cell surface of the engineered T cell is down regulated. The functional protein is preferably protein UL49.5 from EHV-1.

The first aspect of the disclosure also provides a nucleic acid molecule, wherein the nucleic acid molecule is selected from: (1) a nucleic acid molecule containing a coding sequence of a chimeric antigen receptor and a coding sequence of a functional protein capable of regulating the expression of HLA class I molecules on the cell surface; and (2) the complementary sequence of the nucleic acid molecule of (1).

In one or more embodiments, the chimeric antigen receptor and the functional protein are as described in any embodiment of the first aspect of the disclosure.

The first aspect of the disclosure also provides a nucleic acid construct, wherein the nucleic acid construct contains the nucleic acid molecule according to any embodiment of the first aspect of the disclosure.

In one or more embodiments, the nucleic acid construct contains an expression frame of the chimeric antigen receptor and an expression frame of the functional protein; or the nucleic acid construct is an expression frame, wherein the coding sequence of the chimeric antigen receptor and the coding sequence of the functional protein are in the expression frame.

In one or more embodiments, the nucleic acid construct is a cloning vector or an expression vector.

The first aspect of the disclosure also provides a lentivirus containing the nucleic acid construct according to any embodiment of the first aspect of the disclosure.

The first aspect of the disclosure also provides a host cell containing a nucleic acid molecule or nucleic acid construct or lentivirus according to any embodiment of the first aspect of the disclosure.

The first aspect of the disclosure also provides a pharmaceutical composition, wherein the pharmaceutical composition contains the engineered T cells according to any embodiment of the first aspect of the disclosure.

The first aspect of the disclosure also provides use of the functional protein or its coding sequence described in any embodiment of the first aspect of the disclosure in the preparation of the engineering modified T cells with regulated expression of HLA class I molecules on the cell surface, or in the preparation of engineered T cells for cancer treatment, wherein the expression of HLA-A and HLA-B and the expression of HLA-E on the cell surface of the engineering modified T cells are differentially regulated, or the expression of HLA-A, HLA-B and HLA-E on the cell surface of the engineered T cells is down regulated.

In one or more embodiments, differential regulation is that the expression of HLA-A and HLA-B on the cell surface of the engineered T cells is down regulated, but the expression of HLA-E is not down regulated.

In one or more embodiments, the expression level of HLA-A and/or HLA-B molecules on the surface of the engineered T cells is 5-50%, 10-50%, 10-45%, 10-40%, 15-50%, 20-50%, or 20-45% of that of the T cells that do not express the functional protein.

In one or more embodiments, the expression level of HLA-E molecules on the surface of the engineered T cells is 65-100%, 70-100%, 75-100%, 80-100%, 85-100%, 90-100%, 95-100% of that of the T cells that do not express the functional protein.

In one or more embodiments, the functional protein is selected from: protein US 11 from HCMV and protein k5 from KSHV, and the expression of HLA-A and HLA-B and the expression of HLA-E on the cell surface of the engineered T cells are differentially regulated. Preferably, on the cell surface of the engineered T cells, the expression of HLA-A and HLA-B are down regulated, but the expression of HLA-E is not down regulated.

In one or more embodiments, the functional protein is selected from: protein US6 from HCMV, protein UL49.5 from BHV-1 and protein UL49.5 from EHV-1. The genotype of HLA-E of the engineered T cell is 01:03/01:03, and the expression of HLA-A and HLA-B and the expression of HLA-E on the cell surface of the engineered T cell are differentially regulated. Preferably, on the cell surface of the engineered T cell, the expression of HLA-A and HLA-B is down regulated, but the expression of HLA-E is not down regulated. The functional protein is preferably protein UL49.5 from EHV-1.

In one or more embodiments, the functional protein is selected from: protein US6 from HCMV, protein UL49.5 from BHV-1 and protein UL49.5 from EHV-1. The genotype of HLA-E of the engineered T cell is 01:01/01:01, and the expression of HLA-A, HLA-B and HLA-E on the cell surface of the engineered T cell is down regulated. The functional protein is preferably protein UL49.5 from EHV-1.

The disclosure also provides a method for inhibiting the expression of HLA class I molecules on the surface of T cells. The method comprises the step of expressing the functional protein described in any embodiment herein in T cells, wherein on the cell surface of T cells expressing functional protein, the expression of HLA-A and HLA-B and the expression of HLA-E are differentially regulated, or the expression of HLA-A, HLA-B and HLA-E on the cell surface of T cells expressing functional protein is down regulated.

In one or more embodiments, differential regulation is that on the cell surface of the engineered T cells, the expression of HLA-A and HLA-B is down regulated, but the expression of HLA-E is not down regulated.

In one or more embodiments, the expression level of HLA-A and/or HLA-B molecules on the surface of the engineered T cells is 5-50%, 10-50%, 10-45%, 10-40%, 15-50%, 20-50%, or 20-45% of the T cells that do not express the functional protein.

In one or more embodiments, the expression level of HLA-E molecules on the surface of the engineered T cells is 65-100%, 70-100%, 75-100%, 80-100%, 85-100%, 90-100%, 95-100% of the T cells that do not express the functional protein.

The second aspect of the disclosure provides a CAR-T cell, which contains a chimeric antigen receptor specifically targeting tumor antigen and a functional protein capable of regulating the expression of HLA class I molecules on the cell surface.

In one or more embodiments, the expression level of HLA class I molecules on the cell surface of the CAR-T cells is less than 50% of the control CAR-T cells that express the same chimeric antigen receptor but do not express the functional protein.

In one or more embodiments, the CAR-T cell contains a coding sequence of the chimeric antigen receptor and a coding sequence of the functional protein; preferably, the CAR-T cell contains an expression frame of the chimeric antigen receptor and an expression frame of the functional protein, or the coding sequence of the chimeric antigen receptor and the coding sequence of the functional protein are in the same expression frame.

In one or more embodiments, the chimeric antigen receptor specifically binds one or more tumor antigens selected from the group consisting of EGFRvIII, mesothelin, gD2, Tn antigen, sTn antigen, Tn-O-glycopeptide, sTn-O-glycopeptide, PSMA, CD97, TAG72, CD44v6, CEA, EpCAM, KIT, IL-13RA2, Leguman, GD3, CD171, IL-11RA, PSCA, MAD-CT-1, MAD-CT-2, VEGFR2, LewisY, CD24, PDGFR-β, SSEA-4, folate receptor α, ERBB, HER2/neu, MUC1, EGFR, NCAM, Ephrin B2, CAIX, LMP2, SLE, HMWMAA, o-acetyl-GD2, folate receptor β, TEM1/CD248, TEM7R, FAP, Legumain, HPV E6 or E7, ML-IAP, CLDN6, TSHR, GPRC5d, ALK, polysialic acid, FOS related antigen, neutrophil elastase, TRP-2, CYP1B1, sperm protein 17, β Human chorionic gonadotropin, AFP, thyroglobulin, PLAC1, globoH, RAGE1, MN-CA IX, human telomerase reverse transcriptase, intestinal carboxylesterase, mut HSP 70-2, NA-17, NY-BR-1, UPK2, HAVCR1, ADRB3, PANX3, NY-ESO-1, GPR20, Ly6k, OR51E2, TARP, GFRα4 and the polypeptide fragments of any of these antigens presented on MHC, as well as CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79A, CD79B, CD80, CD81, CD82, CD83, CD84, CD85, CD86, CD123, CD135, CD138, CD179, CD269, Flt3, ROR1, BCMA, FcRn5, FcRn2, CS-1, CXCR4, CXCR5, CXCR7, IL-7/3R, IL-7/4/3R, and IL4R.

In one or more embodiments, the functional protein capable of regulating the expression of HLA class I molecules on the cell surface is selected from the group consisting of HSV, BHV-1, EHV-1/4, PRV, HSV-1/2, VZV, EBV, HCMV, MCMV, RhCMV, HHV-6/7, KSHV, MHV-68, the functional protein capable of directly target-degrading HLA class I molecules in cowpox virus and adenovirus, the functional protein capable of directly target-degrading HLA class I molecules through TAP protein, and the functional protein capable of down regulating the expression of HLA class I molecules by lysosomes.

In one or more embodiments, the functional protein is selected from proteins US11 and US6 from HCMV, protein UL49.5 from BHV-1, protein UL49.5 from EHV-1 and protein k5 from KSHV.

In one or more embodiments, the regulation of the expression of HLA class I molecules on the cell surface comprises: down regulating the expression of HLA-A, HLA-B and HLA-E on the cell surface, or differentially regulating the expression of HLA-A and HLA-B and the expression of HLA-E on the cell surface.

In one or more embodiments, differential regulation is that on the cell surface of CAR-T cells, the expression of HLA-A and HLA-B is down regulated, but the expression of HLA-E is not down regulated.

In one or more embodiments, the expression of HLA-A and HLA-B and the expression of HLA-E on the cell surface of CAR-T cells are differentially regulated, and the functional protein is selected from: protein US 11 from HCMV and protein k5 from KSHV. Preferably, on the cell surface of CAR-T cells, the expression of HLA-A and HLA-B is down regulated, but the expression of HLA-E is not down regulated.

In one or more embodiments, the functional protein is selected from: protein US6 from HCMV, protein UL49.5 from BHV-1 and protein UL49.5 from EHV-1. The genotype of HLA-E of CAR-T cells is 01:03/01:03, and the expression of HLA-A and HLA-B and the expression of HLA-E on the cell surface of CAR-T cells are differentially regulated. Preferably, on the cell surface of the engineered T cells, the expression of HLA-A and HLA-B is down regulated, but the expression of HLA-E is not down regulated. The functional protein is preferably UL49.5 from EHV-1.

In one or more embodiments, the functional protein is selected from: protein US6 from HCMV, protein UL49.5 from BHV-1 and protein UL49.5 from EHV-1. The genotype of HLA-E of the engineered T cell is 01:01/01:01, and the expression of HLA-A, HLA-B and HLA-E on the cell surface of the engineered T cell is down regulated. The functional protein is preferably protein UL49.5 from EHV-1.

In one or more embodiments, the expression level of HLA-A and/or HLA-B molecules on the cell surface of CAR-T cells is 5-50%, 10-50%, 10-45%, 10-40%, 15-50%, 20-50%, or 20-45% of T cells that do not express the functional protein.

In one or more embodiments, the expression level of HLA-E molecules on the cell surface of CAR-T cells is 65-100%, 70-100%, 75-100%, 80-100%, 85-100%, 90-100%, 95-100% of T cells that do not express the functional protein.

The disclosure provides a CAR-T cell, which contains a chimeric antigen receptor specifically targeting tumor antigen and a functional protein that can differentially regulate the expression of HLA-A, HLA-B and HLA-E on the cell surface. Preferably, the functional protein is selected from: protein US 11 from HCMV and protein k5 from KSHV. Preferably, on the cell surface of the CAR-T cell, the expression of HLA-A and HLA-B is down regulated, but the expression of HLA-E is not down regulated.

The present disclosure also provides a nucleic acid molecule selected from:
(1) A nucleic acid molecule containing a coding sequence of chimeric antigen receptors and a coding sequence of functional proteins capable of regulating the expression of HLA class I molecules on the cell surface; and
(2) the complementary sequence of the nucleic acid molecule of (1).

In one or more embodiments, the chimeric antigen receptor and the functional protein are as described in any embodiment of the second aspect herein.

The present disclosure also provides a nucleic acid construct comprising the nucleic acid molecule described in any embodiment of the second aspect of the disclosure.

In one or more embodiments, the nucleic acid construct contains an expression frame of the chimeric antigen receptor and an expression frame of the functional protein; or the nucleic acid construct is an expression frame, wherein the coding sequence of the chimeric antigen receptor and the coding sequence of the functional protein are in the expression frame.

In one or more embodiments, the nucleic acid construct is a cloning vector or an expression vector.

The present disclosure also provides a lentivirus containing the nucleic acid construct described in the second aspect of the disclosure.

The present disclosure also provides a host cell containing the nucleic acid molecule, nucleic acid construct or lentivirus described in the second aspect of the disclosure.

The present disclosure also provides a pharmaceutical composition containing CAR-T cells according to any embodiment of the second aspect of the disclosure.

The present disclosure also provides use of the functional protein or its coding sequence according to the second aspect of the disclosure in the preparation of CAR-T cells with regulated expression of HLA class I molecules on the cell surface, or in the preparation of CAR-T cells for cancer treatment.

In one or more embodiments, the regulation of the expression of HLA class I molecules on the cell surface comprises: down regulating the expression of HLA-A, HLA-B and HLA-E on the cell surface, or differentially regulating the expression of HLA-A and HLA-B and the expression of HLA-E on the cell surface.

In one or more embodiments, differential regulation is that on the cell surface of CAR-T cells, the expression of HLA-A and HLA-B is down-regulated, but the expression of HLA-E is not down regulated.

In one or more embodiments, the functional protein is selected from: protein US 11 from HCMV and protein k5 from KSHV, and the expression of HLA-A and HLA-B and the expression of HLA-E on the cell surface of CAR-T cells are differentially regulated. Preferably, on the cell surface of CAR-T cells, the expression of HLA-A and HLA-B are down regulated, but the expression of HLA-E is not down regulated.

In one or more embodiments, the functional protein is selected from: protein US6 from HCMV, protein UL49.5 from BHV-1 and protein UL49.5 from EHV-1. The genotype of HLA-E of CAR-T cells is 01:03/01:03, and the expression of HLA-A and HLA-B on the cell surface of CAR-T cells and the expression of HLA-E are differentially regulated. Preferably, on the cell surface of CAR-T cells, the expression of HLA-A and HLA-B is down regulated, but the expression of HLA-E is not down regulated. The functional protein is preferably UL49.5 from EHV-1.

In one or more embodiments, the functional protein is selected from: protein US6 from HCMV, protein UL49.5 from BHV-1 and protein UL49.5 from EHV-1. The genotype of HLA-E of CAR-T cells is 01:01/01:01, and the expression of HLA-A, HLA-B and HLA-E on the cell surface of CAR-T cells is down regulated. The functional protein is preferably protein UL49.5 from EHV-1.

In one or more embodiments, the expression level of HLA-A and/or HLA-B molecules on the surface of the engineered T cells is 5-50%, 10-50%, 10-45%, 10-40%, 15-50%, 20-50%, or 20-45% of the T cells that do not express the functional protein.

In one or more embodiments, the expression level of HLA-E molecules on the surface of the engineered T cells is 65-100%, 70-100%, 75-100%, 80-100%, 85-100%, 90-100%, 95-100% of the T cells that do not express the functional protein.

The disclosure also provides use of a functional protein or its coding sequence in the preparation of CAR-T cells of which the expression of HLA-A and HLA-B and the expression of HLA-E expression on the cell surface are differentially regulated, or in the preparation of CAR-T cells for cancer treatment. The functional protein is selected from: protein US 11 from HCMV and protein k5 from KSHV. In one or more embodiments, differential regulation is that on the cell surface of CAR-T cells, the expression of HLA-A and HLA-B is down regulated, but the expression of HLA-E is not down regulated.

The disclosure also provides use of a functional protein or its coding sequence in the preparation of CAR-T cells of which the expression of HLA-A and HLA-B and the expression of HLA-E on the cell surface are differentially regulated, or in the preparation of CAR-T cells for cancer treatment. The HLA-E genotype of CAR-T cells is 01:03/01:03, and the functional protein is selected from: protein US6 from HCMV, protein UL49.5 from BHV-1 and protein UL49.5 from EHV-1. The functional protein is preferably protein UL49.5 from EHV-1. In one or more embodiments, differential regulation is that on the cell surface of CAR-T cells, the expression of HLA-A and HLA-B is down regulated, but the expression of HLA-E is not down regulated.

The disclosure also provides use of a functional protein or its coding sequence in the preparation of CAR-T cells with down regulated expression of HLA-A, HLA-B and HLA-E on the cell surface, or in the preparation of CAR-T cells for cancer treatment. The genotype of HLA-E of CAR-T cells is 01:01/01:01, and the functional protein is selected from: protein US6 from HCMV, protein UL49.5 from BHV-1 and protein UL49.5 from EHV-1. The functional protein is preferably protein UL49.5 from EHV-1.

The second aspect of the disclosure also provides a method for inhibiting the expression of HLA class I molecules on the surface of CAR-T cells, comprising the step of simultaneously expressing CAR and functional protein according to any embodiment herein in T cells.

In one or more embodiments, on the cell surface of CAR-T cells, the expression of HLA-A and HLA-B and the expression of HLA-E are differentially regulated, and the functional protein is selected from: protein US 11 from HCMV and protein k5 from KSHV. Preferably, on the cell surface of CAR-T cells, the expression of HLA-A and HLA-B is down regulated, but the expression of HLA-E is not down regulated.

In one or more embodiments, the genotype of HLA-E of CAR-T cells is 01:03/01:03, and the expression of HLA-A and HLA-B and the expression of HLA-E on the cell surface of the T cells are differentially regulated. The functional protein is selected from: protein US6 from HCMV, protein UL49.5 from BHV-1 and protein UL49.5 from EHV-1. The functional protein is preferably protein UL49.5 from EHV-1. Preferably, on the cell surface of CAR-T cells, the expression of HLA-A and HLA-B is down regulated, but the expression of HLA-E is not down regulated.

In one or more embodiments, the genotype of HLA-E of CAR-T cells is 01:01/01:01, and the expression of HLA-A, HLA-B and HLA-E on the cell surface of the T cells is down regulated. The functional protein is selected from: protein US6 from HCMV, protein UL49.5 from BHV-1 and protein UL49.5 from EHV-1. The functional protein is preferably protein UL49.5 from EHV-1.

The disclosure also provides a method for differentially regulating the expression of HLA-A, HLA-B and HLA-E on the surface of CAR-T cells. The method comprises the steps of simultaneously expressing CAR of any embodiment of this disclosure and a functional protein selected from: protein US11 from HCMV and protein k5 from KSHV. In one or more embodiments, differential regulation is that on the cell surface of CAR-T cells, the expression of HLA-A and HLA-B is down regulated, but the expression of HLA-E is not down regulated.

The disclosure also provides a method for differentially regulating the expression of HLA-A, HLA-B and HLA-E on the surface of CAR-T cells. The method comprises the steps of simultaneously expressing CAR of any embodiment of this disclosure and a functional protein selected from the following functional proteins in T cells: protein US6 from HCMV, protein UL49.5 from BHV-1 and protein UL49.5 from EHV-1. The genotype of HLA-E of the T cell is 01:03/01:03. The functional protein is preferably protein UL49.5 from EHV-1. In one or more embodiments, differential regulation is that on the cell surface of CAR-T cells, the expression of HLA-A and HLA-B is down-regulated, but the expression nof HLA-E is not down regulated.

The disclosure also provides a method for inhibiting the expression of HLA-A, HLA-B and HLA-E on the surface of CAR-T cells. The method comprises the steps of simultaneously expressing CAR of any embodiment of this disclosure and a functional protein selected from: protein US6 from HCMV, protein UL49.5 from BHV-1 and protein UL49.5 from EHV-1. The genotype of HLA-E of the T cells is 01:01/01:01. The functional protein is preferably protein UL49.5 from EHV-1.

### Description of drawings

Fig. 1: activated T cells were transfected with lentivirus vector in each group. T cells were cultured in vitro until Day 8. The average fluorescence intensity of HLA class I molecules on the surface of CAR19⁺ T cells was detected by flow cytometry.
Fig. 2: on Day 9 of T cell culture in vitro, T cells in each group were stimulated with target cell K562-CD19, with an effect target ratio of 10:1. After repeated stimulation for 2 days, the average fluorescence intensity of HLA class I molecules in CAR19⁺ cell population of T cells in each group was detected by flow cytometry, wherein five carrier molecules PCTL200, PCTL201, PCTL205, PCTL206 and PCTL213 have the ability to prepare CAR-T cells with down regulated expression of HLA class I molecules.
Fig. 3: flow cytometric analysis of the proportion of whole blood lymphocytes. 1.24x10⁸ PBMCs were isolated by Ficoll density gradient centrifugation of 100 ml peripheral blood. According to flow cytometry, CD3+ T accounted for 62.4% of leukocytes, with a CD4 + T / CD8 + T ratio of 1.2 and 24-hour activation efficiency of 68.9%.
Fig. 4: expansion multiple of the cells of each group expanded in vitro for 8 days. The cells in each group were expanded in vitro for 8 days, and the expansion multiple was significantly higher than that in PCTL135 group.
Fig. 5: average fluorescence intensity of HLA class I molecules of T cells in each group. By flow cytometry, the average fluorescence intensity of HLA class I molecules of CAR positive cells in each group were significantly down regulated, especially in PCTL206 group.
Fig. 6: killing efficiency of T cells in each group. The cells in each group were used for cytotoxicity experiment in vitro, and the killing efficiency was greater than 90% at the effective target ratio of 5:1, which was not significantly different from the control group.
Fig. 7: analysis of differentiation phenotype of T cells in each group. The differentiation phenotype of the cells in each group was detected, and more than 65% of T cells were showed as T-naïve group, which was not significantly different from control group 135.
Fig. 8: average fluorescence intensity of HLA class I molecules of T cells in each group stimulated by target cell K562-CD19. After cultured in vitro to Day 9, T cells in each group were stimulated with target cell K526-CD19. After stimulation, HLA class I molecules of CAR positive cell population in each group were detected by flow cytometry. It was found that the average fluorescence intensity was significantly lower than that in the control group.
Fig. 9: average fluorescence intensity of various HLA class I molecules in CAR19⁺ T cell population with HLA-E genotype of heterozygous 01:01/01:03 by flow cytometry.
Fig. 10: average fluorescence intensity of various HLA class I molecules in CAR19⁺ T cell population with HLA-E genotype of homozygous 01:01/01:01 by flow cytometry.
Fig. 11: average fluorescence intensity of various HLA class I molecules in CAR19⁺ T cell population with HLA-E genotype of homozygous 01:03/01:03 by flow cytometry.

### Embodiments

It should be understood that within the scope of the disclosure, the above technical features of the disclosure and the technical features specifically described below (according to the examples) can be combined with each other to form a preferred technical solution.

Because gene editing technology can efficiently and completely eliminate the expression of one or more targeted genes at the genomic level, they have quickly become the mainstream technical means for preparing allogeneic CAR-T to completely knockout the expression of TCR and HLA class I molecules of allogeneic CAR-T cells, which is expected to effectively prevent GVHD and host rejection in clinic.

The basic principle of preparing allogeneic T cells by gene editing is: producing site-specific double strand break (DSB) at specific positions of the genome (such as TCR gene and HLA class I molecular chaperone b2m gene), and then repairing it by non-homologous end joining (NHEJ) or homology directed recombination repair (HDR), thereby resulting in the complete deletion of targeted genes (such as TCR and b2m genes), which is designed to fundamentally avoid GVHD and host immune cell-mediated rejection of allogeneic CAR-T cells.

However, so far, allogeneic CAR-T cells based on gene editing technology have not shown the same therapeutic advantages as autologous CAR-T cells in clinical trials. In-depth studies have found that allogeneic CAR-T cells produced by gene editing have poor persistence in patients, one of the reasons is that b2m gene knockout leads to the lack of the expression of all HLA class I molecules, especially HLA-A, HLA-B and HLA-E in allogeneic CAR-T cells. The allogeneic CAR-T cells are prone to attack by patient's NK cells, which seriously affects the persistence and efficacy of allogeneic CAR-T in patients (Torikai et al. 2013).

In order to prolong the *in vivo* persistence of reinfused allogeneic CAR-T cells, Precision Biosciences used Arcus genome editing technology to knockout only TCR gene, but did not edit b2m gene (i.e. without changing the expression of HLA class I molecules on the surface of CAR-T cells). Therefore, its allogeneic CAR-T cells express normal HLA class I molecules. After the first and second dose climbing tests, it submitted a clinical amendment of allogeneic CAR-T to FDA in early 2020, applied for enhancing the chemotherapy pretreatment before reinfusion, and applied for significantly increasing the reinfusion dose of allogeneic CAR-T cells in order to achieve the expected therapeutic effect.

Cellectis and Allogene Therapeutics used TALEN gene editing technology to knockout TCR and CD52 genes of donor T cells (i.e. without changing the expression of HLA class I molecules on the surface of CAR-T cells), and then combine it with anti-CD52 monoclonal antibody to utilize its lymph elimination activity in order to inhibit the rejection of both patient's T cells and NK cells to allogeneic CAR-T cells.

Obviously, those skilled in the art are facing an insurmountable dilemma; on one hand, without changing the expression level of HLA class I molecules, the risk of attack by the patient's T cell immune system on CAR-T cells remains; on the other hand, the absolute loss of expression of HLA class I molecules caused by gene editing results in the attack of the patient's NK cells on CAR-T cells; through these studies, those skilled in the art come to a frustrating logical conclusion that regulating the expression of HLA class I molecules cannot eliminate the risk of attack by the patient's immune system on CAR-T cells. Cellectis and Allogene Therapeutics, without changing the expression of HLA class I molecules on the surface of CAR-T cells, removed the attack by both the patient T cells and NK cells on CAR-T cells through the deletion of CD52 gene of CAR-T cells and then use of anti-CD52 antibody, which is an attempt to get out of the dilemma that also further proves this logical conclusion.

In view of these clinical observations and findings, in CN109694854a patent application, Gracell disclosed that on the basis of gene knockout TCR and all HLA class I molecules, the surface expression of HLA-E molecules can be restored by overexpressing HLA-E single chain trimer, so as to avoid the rejection of both host T cells and NK cells to allogeneic CAR-T cells.

After years of in-depth research, the inventor of the disclosure and his research team unexpectedly found that by expressing a functional protein in allogeneic T cells (especially CAR-T cells), the expression level of HLA class I molecules on the cell surface can be differentially regulated while the expression level of HLA-E molecules on the cell surface is maintained or limitedly inhibited; such allogeneic T cells (especially CAR-T cells) can not only avoid GVHD, but also eliminate the risk of attack by patient's immune system (T cells and NK cells) on allogeneic T cells.

HLA class I molecules are composed of classical HLA class I molecules (HLA-A, HLA-B, etc.) and non classical HLA class I molecules (HLA-E, etc.). HLA-E molecules can inhibit the biological activity of NK cells by binding to CD94/NKG2 receptors on the surface of NK cells. The gene of HLA-E in human body contains two alleles 01:01 and 01:03.

The inventor found that when the functional protein is selected from protein US 11 from HCMV and protein k5 from KSHV, the functional protein differentially regulates the expression of HLA-A and HLA-B and the expression of HLA-E. Therefore, the expression of HLA-E molecules is retained on the cell surface in the T cells (especially CAR-T cells) of the disclosure, which can effectively avoid being cleared by the host NK cells, so as to improve the survival and activity of the T cells (especially CAR-T cells).

In addition, the inventor also found that when the functional protein is selected from protein US6 from HCMV, protein UL49.5 from BHV-1 and protein UL49.5 from EHV-1, and the genotype of HLA-E of the T cells (especially CAR-T cells) is 01:03/01:03, the functional protein differentially regulates the expression of HLA-A and HLA-B and the expression of HLA-E on the cell surface of the T cells (especially CAR-T cells). When the functional protein is selected from protein US6 from HCMV, protein UL49.5 from BHV-1 and protein UL49.5 from EHV-1, and the genotype of HLA-E of the T cells (especially CAR-T cells) is 01:01/01:01, the functional protein down regulates the expression of HLA-A, HLA-B and HLA-E on the cell surface of the T cells (especially CAR-T cells). The functional protein is preferably protein UL49.5 from EHV-1.

In some embodiments, the expression levels of HLA-A and HLA-B molecules on the surface of allogeneic T cells (especially CAR-T cells) are 5-50%, 10-50%, 10-45%, 10-40%, 15-50%, or 20-50%, or 20-45% of T cells that do not express the functional protein; the expression levels of HLA-E molecules are 65-100%, 70-100%, 75-100%, 80-100%, 85-100%, 90-100%, 95-100% of T cells that do not express the functional protein.

In some embodiments, allogeneic T cells (especially CAR-T cells) express functional proteins that can differentially regulate the expression levels of HLA-A, HLA-B and HLA-E molecules, including but not limited to:
UL49.5 or those having at least 85%, 86%, 87%, 88%, 89%, 90%, 91%, 92%, 93%, 94%, 95%, 96%, 97%, 98%, or 99% homology with UL49.5; UL49.5 is from BHV-1 (SEQ ID No: 8) or EHV-1/4 (SEQ ID No: 9), which can induce conformational arrest of TAP, or induce conformational arrest and degradation of TAP, or block the binding of ATP and TAP, so as to inhibit the expression of MHC-I molecules on the cell surface. The results were verified on bovine renal cells and equine epidermal cells respectively (Koppers-Lalic et al. 2008);
US6/gp21 (SEQ ID No: 10), or a variant of US6/gp21, which has at least 85%, 86%, 87%, 88%, 89%, 90%, 91%, 92%, 93%, 94%, 95%, 96%, 97%, 98%, or 99% homology with SEQ ID No: 10; US11/gp33 (SEQ ID No: 11), or a variant of US11/gp33, which has at least 85%, 86%, 87%, 88%, 89%, 90%, 91%, 92%, 93%, 94%, 95%, 96%, 97%, 98%, or 99% homology with SEQ ID No: 11; US6/gp21 and US11/gp33 come from HCMV, which can induce conformational changes of TAP to prevent ATP binding and target MHC-I to cause endoplasmic reticulum mediated protein degradation, so as to inhibit the expression of MHC-I molecules on the cell surface. This result is verified on Chinese hamster ovary cells, human myeloid leukemia cells, human lung tumor cells and human astrocytoma cells, respectively (Hewitt et al. 2001, Wiertz et al. 1996);
kK5/MIR2 (SEQ ID NO:12), or a variant of kK5/MIR2, which has at least 85%, 86%, 87%, 88%, 89%, 90%, 91%, 92%, 93%, 94%, 95%, 96%, 97%, 98%, or 99% homology with SEQ ID No: 12; kK5/MIR2 comes from KSHV, which makes MHC-I ubiquitinated to be internalized and degraded by lysosomes, thereby inhibiting the expression of MHC-I molecules on the cell surface. This result has been verified on human cervical cancer cells (Coscoy et al. 2005).

Although the above functions of HLA class I down-regulating proteins partially inhibit the expression of HLA class I molecules in specific cell types of specific species or natural susceptible cells, it remains unknown whether to the expression of HLA class I molecules is down regulated in target cell types (such as T cells) of target species (such as human), and even in the situation wherein chimeric antigen receptor (CAR) is expressed in T cells at the same time. In one or more embodiments, mK3 of MHV-68, US3/gp23 of hCMV, CPXV012 of cowpox virus, rh178/VIHCE of RhCMV and E3-19K of adenovirus failed to show the ability to down regulate HLA class I molecules in T cells. In one or more embodiments, the inventor found that a plurality of cross species viruses showed an unexpected effect of down regulating HLA class I molecules in human T cells, such as EHV-1 (equine Rhinopneumonia virus) UL49.5, etc.

The disclosure regulates HLA class I molecules on the cell surface by expressing HLA class I associated functional protein and preparing engineered T cells capable of inhibiting the expression of HLA class I molecules on the cell surface; one example of the engineered T cells is allogeneic CAR-T cells. In the engineered T cells prepared by the method of the disclosure, the expression of HLA class I molecules is not completely inhibited, and these T cells still express a certain amount of HLA class I molecules on the cell surface, so it can well avoid the attack by receptor's NK cells and solve the rejection reaction of receptors. Preferably, by flow cytometry, the expression level of HLA class I molecules in the engineered T cells of the disclosure is less than 80%, preferably less than 60%, more preferably less than 50%, more preferably less than 30%, more preferably less than 25% of the control engineered T cells wherein the functional protein is not expressed. In some embodiments, by flow cytometry, the expression level of HLA class I molecules in the engineered T cells of the disclosure is 10-50%, such as 15-50% of the control engineered T cells wherein the functional protein is not expressed. In some embodiments, even after stimulation with the same target cell, the expression level of HLA class I molecules in the engineered T cells of the disclosure is still 10-50%, such as 15-50% of the control engineered T cells wherein the functional protein is not expressed.

The engineered T cells may be CAR-T cells. Therefore, the disclosure provides a class of CAR-T cells, which contain nucleic acid molecules encoding chimeric antigen receptors (CARs) targeting tumor antigens of interest and nucleic acid molecules encoding functional proteins capable of regulating the expression of HLA class I molecules on the cell surface. In this disclosure, suitable T cells can be various T cells known in the art, especially various T cells commonly used in cellular immunotherapy, including but not limited to peripheral blood T lymphocytes, cytotoxic T cells, helper T cells, inhibitory/regulatory T cells, γδT cells, cytokine induced killer cells and tumor infiltrating lymphocytes, as well as mixtures of any one or more of above cells. In this disclosure, CAR-T cells refer to T cells that express at least chimeric antigen receptors.

In this disclosure, chimeric antigen receptor has a well-known meaning in the art. It is an artificially modified receptor, which can anchor specific molecules (such as antibodies) that recognize tumor cell surface antigens on immune cells (such as T cells), so that the immune cells can recognize tumor antigens and kill tumor cells.

The suitable chimeric antigen receptor in this disclosure can be various CARs well known in the art. Generally, CAR contains a polypeptide binding to tumor antigen, hinge region, transmembrane region and intracellular signaling region in turn. The polypeptide binding to tumor antigen can be a natural polypeptide or synthetic polypeptide; preferably, the synthetic polypeptide is a single chain antibody or Fab fragment.

In this disclosure, tumor antigens of interest include, but are not limited to, solid tumor antigens, myeloid tumor antigens, and antigens of non-B-cell lineage blood tumors. Suitable solid tumor antigens include but are not limited to EGFRvIII, mesothelin, gD2, Tn antigen, sTn antigen, Tn-O-glycopeptide, sTn-O-glycopeptide, PSMA, CD97, TAG72, CD44v6, CEA, EpCAM, KIT, IL-13RA2, Leguman, GD3, CD171, IL-11RA, PSCA, MAD-CT-1, MAD-CT-2, VEGFR2, LewisY, CD24, PDGFR-β, SSEA-4, folate receptor α, ERBB, HER2/neu, MUC1, EGFR, NCAM, Ephrin B2, CAIX, LMP2, SLE, HMWMAA, o-acetyl-GD2, folate receptor β, TEM1/CD248, TEM7R, FAP, Legumain, HPV E6 or E7, ML-IAP, CLDN6, TSHR, GPRC5d, ALK, polysialic acid, FOS related antigen, neutrophil elastase, TRP-2, CYP1B1, sperm protein 17, β Human chorionic gonadotropin, AFP, thyroglobulin, PLAC1, globoH, RAGE1, MN-CA IX, human telomerase reverse transcriptase, intestinal carboxylesterase, mut HSP 70-2, NA-17, NY-BR-1, UPK2, HAVCR1, ADRB3, PANX3, NY-ESO-1, GPR20, Ly6k, OR51E2, TARP, GFRα4 and the polypeptide fragments of any of these antigens presented on MHC. Suitable B cell antigens include, but are not limited to, CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79A, CD79B, CD80, CD81, CD82, CD83, CD84, CD85, CD86, CD123, CD135, CD138, CD179, CD269, FLT3, ROR1, BCMA, FcRn5, FcRn2, CS-1, CXCR4, CXCR5, CXCR7, IL-7/3R, IL7/4/3R, and IL4R.

In a preferred embodiment, the polypeptide binding to the tumor antigen of the disclosure is a single chain antibody specifically binding to any of the above tumor antigens. In this disclosure, single chain antibody (scFv) refers to an antibody fragment with antigen binding ability formed by hinge connection of an amino acid sequence of antibody light chain variable region (VL region) and an amino acid sequence of heavy chain variable region (VH region). scFvs of interest may be from antibodies of interest. Antibodies of interest may be human antibodies, including human murine chimeric antibodies and humanized antibodies. The antibody can be secretory or membrane anchored, preferably membrane anchored. In this disclosure, specific binding refers to the reaction between an antibody or its antigen binding fragment and its target antigen. In some embodiments, an antibody that specifically binds to an antigen (or an antibody that is specific to an antigen) means that the antibody binds to the antigen with an affinity (KD) of less than about 10⁻⁵M, such as less than about 10⁻⁶M, 10⁻⁷M, 10⁻⁸M, 10⁻⁹M or 10⁻¹⁰M or less.

A single chain antibody may contain a heavy chain variable region and a light chain variable region of the antibody of interest, or consist of a heavy chain variable region, a light chain variable region and an optional linker. The heavy chain variable region and the light chain variable region can be connected by a well-known linker. In this disclosure, the linker or hinge is a polypeptide fragment connecting different proteins or polypeptides, of which the purpose is to keep the connected proteins or polypeptides in their respective spatial conformations, so as to maintain the function or activity of proteins or polypeptides. Exemplary linkers include linkers containing G and/or S, and Furin 2A peptide (F2A). The length of the linker can be 3-25 amino acid residues, such as 3-15, 5-15, 10-20 amino acid residues. In some embodiments, the linker sequence is a polyglycine linker sequence. The number of glycine in the linker sequence is not particularly limited, usually is 2-20, such as 2-15, 2-10 and 2-8. In addition to glycine and serine, the linker may also contain other known amino acid residues, such as alanine (A), leucine (L), threonine (T), glutamate (E), phenylalanine (F), arginine (R), glutamine (Q), etc. The linker length is usually 15-20 amino acids. In some embodiments, the linker is (GGGS)ₙ, and n is an integer of 1-5.

In some embodiments, the tumor antigen of interest is CD19, and the scFv of interest is a scFv that specifically binds to CD19. The amino acid sequence of an exemplary scFv that specifically binds to CD 19 is shown as amino acid residues 23-267 of SEQ ID No: 2, wherein the heavy chain variable region and the light chain variable region are connected by a linker sequence containing G and S.

Other parts contained in CAR, such as hinge region, transmembrane region and intracellular signaling region, can be hinge region, transmembrane region and intracellular signaling region conventionally used to construct various CARs.

In this disclosure, the hinge region refers to the region between the functional regions of immunoglobulin heavy chain CH1 and CH2, which is rich in proline, does not form α-helix, and is easy to stretch and twist to a certain extent, which is conducive to the complementary binding between the antigen binding site of the antibody and the antigen epitope. The suitable hinge region in this disclosure can be selected from CD8 extracellular hinge region, IgG1 FC CH2CH3 hinge region, IgD hinge region, CD28 extracellular hinge region, IgG4 FC CH2CH3 hinge region and CD4 extracellular hinge region. In some embodiments, CD8α hinge region is used herein.

In this disclosure, the transmembrane region can be selected from one or more of CD28 transmembrane region, CD8 transmembrane region and CD3ζ transmembrane region, CD134 transmembrane region, CD137 transmembrane region, ICOS transmembrane region and DAP10 transmembrane region. Preferably, the transmembrane region of the chimeric antigen receptor used herein is CD8 transmembrane region. The amino acid sequences of the exemplary hinge region and transmembrane region can be shown as amino acid residues 268-336 of SEQ ID No: 2.

In this disclosure, the intracellular signal region can be selected from any one or more intracellular signaling regions of CD28, CD134/OX40, CD137/4-1BB, LCK, ICOS, DAP10 and CD3ζ and Fc310, preferably 4-1BB intracellular signaling region and CD3ζ intracellular signaling region. The amino acid sequence of the exemplary intracellular signaling region in this disclosure can be shown as amino acid residues 337-490 of SEQ ID No: 2.

The chimeric antigen receptor may also include a signal peptide. A signal peptide is a short peptide chain (5-30 amino acids in length) that guides the transfer of newly synthesized proteins to the secretory pathway. It often refers to the N-terminal amino acid sequence used to guide the transmembrane transfer (localization) of proteins in the newly synthesized peptide chain. The signal peptide can be a membrane protein signal peptide, such as CD8 signal peptide, CD28 signal peptide and CD4 signal peptide. An exemplary signal peptide amino acid sequence may be shown as amino acid residues 1-22 of SEQ ID No: 2.

Therefore, the amino acid sequence of the chimeric antigen receptor of the disclosure, from the N-end to the C-end, is usually an optional signal peptide, a single chain antibody targeting the heavy chain antigen of interest, a hinge region, a transmembrane region and an intracellular signaling region. The amino acid sequence of an exemplary chimeric antigen receptor may be shown as amino acid residues 23-490 of SEQ ID No: 2, or as amino acid residues 1-490 of SEQ ID No: 2.

The above parts forming the chimeric antigen receptor herein, such as signal peptide, light chain variable region and heavy chain variable region of single chain antibody, hinge region, transmembrane region and intracellular signaling region, can be directly connected with each other, or can be connected through linker sequences well known in the art, such as G and S-containing linker sequences described above.

In this disclosure, the functional protein capable of regulating the expression of HLA class I molecules on the cell surface can be a viral protein, preferably a protein from a virus whose natural host cell is not a T cell. Although HIV-1 is the natural host of T cells, and HIV-1 nef virus protein can down regulate the expression of HLA class I molecules on the surface of T cells, the introduction of HIV-1 nef protein into lentivirus to prepare CAR-T will cause RCL (replication competent lentivirus). Therefore, the disclosure preferably uses viral proteins such as those from HSV, BHV-1, EHV-1/4, PRV, HSV-1/2, VZV, EBV, HCMV, MCMV, RhCMV, HHV-6/7, KSHV, MHV-68, cowpox virus and adenovirus, including but not limited to: UL41/VHS protein from HSV, UL49.5 from BHV-1, EHV-1/4 or PRV, ICP47 from HSV-1/2, ORF66 from VZV, EBNA1, BNLF2a, BGLF5 and BILF1 from EBV, US2/gp24, US3/gp23, US6/gp21, US10 and US11/gp33 from hCMV, m4/gp34, m6/gp48, m27 and m152/gp40 from mCMV, rh178/VIHCE from RhCMV, U21 and LANA1 from HHV-6/7, ORF37/SOX, kK3/MIR1 and kK5/MIR2 from KSHV, mK3 from MHV-68, CPXV012 and CPXV203 from cowpox virus, and E3-19K from adenovirus.

In some embodiments, the functional protein that regulates HLA class I class proteins on the cell surface is selected from: protein US 11 from HCMV and k5 from KSHV, resulting in differential regulation of the expression of HLA-A and HLA-B and the expression of HLA-E on the cell surface of CAR-T cells.

In some embodiments, the functional protein that regulates HLA class I molecules on the cell surface is selected from: protein US6 from HCMV, protein UL49.5 from BHV-1 and protein UL49.5 from EHV-1. For CAR-T cells that express these functional proteins with HLA-E genotype of 01:03/01:03, the expression of HLA-A, HLA-B and HLA-E on the cell surface are differentially regulated. For CAR-T cells that express these functional proteins with HLA-E genotype 01:01/01:01, the expression of HLA-A, HLA-B and HLA-E on the cell surface were down regulated. The functional protein is preferably protein UL49.5 from EHV-1.

The preferred functional protein is a functional protein that can directly target degrade MHC I, or a functional protein that can down regulate the expression of HLA class I molecules through TAP proteins (such as inhibiting TAP, including preventing TAP proteins from binding ATP and/or inducing TAP protein degradation), or a functional protein that can down regulate the expression of HLA class I molecules through lysosomes. Exemplary preferred proteins include, but are not limited to, viral proteins from HCMV, such as US 11 and US6, viral proteins from BHV-1, such as UL49.5, viral proteins from EHV-1, such as UL49.5, and viral proteins from KSHV, such as k5. In some embodiments, the disclosure uses UL49.5 from EHV-1, and its amino acid sequence can be shown as amino acid residues 516-615 of SEQ ID No: 2.

The viral protein can be connected to the CAR of the disclosure through a linker commonly used in the art. For example, in some embodiments, the linker is a conventional F2A sequence. The amino acid sequence of an exemplary F2A may be shown as amino acid residues 494-515 of SEQ ID No: 2. F2A can also be connected to CAR through conventional linkers containing G and S.

The nucleic acid molecule of the disclosure can be in the form of DNA or RNA. DNA forms include cDNA, genomic DNA or synthetic DNA. DNA can be single stranded or double stranded. The nucleic acid molecule of the disclosure can be the coding sequence of CAR and the coding sequence of functional protein capable of regulating the expression of HLA class I molecules on the cell surface, or the expression frame of CAR and the expression frame of the functional protein. In this disclosure, the coding sequence refers to the part of the nucleic acid sequence that directly determines the amino acid sequence of its protein product (such as CAR, single chain antibody, hinge region, transmembrane region, intracellular signaling region, viral protein or its fusion protein, etc.). The boundary of the coding sequence is usually determined by the ribosome binding site immediately upstream of the mRNA 5' open reading frame (for prokaryotic cells) and the transcription termination sequence immediately downstream of the mRNA 3' open reading frame. Coding sequences may include, but are not limited to, DNA, cDNA and recombinant nucleic acid sequences. In this disclosure, the expression frame refers to the complete elements required to express the gene of interest, including promoter, gene coding sequence and polyA tailing signal sequence. The nucleic acid molecule described herein can be two independent nucleic acid molecules, respectively containing the coding sequence of CAR and the coding sequence of the functional protein, such as the expression frame of CAR and the expression frame of functional protein; alternatively, the coding sequence containing CAR and the coding sequence of the functional protein can be connected in one nucleic acid molecule through a linker, for example, the coding sequence of CAR and the coding sequence of the functional protein are in the same expression frame, or the two expression frames are connected into the same nucleic acid molecule through a suitable linker. In some embodiments, the nucleic acid molecule of the disclosure is a nucleic acid molecule in which the coding sequence of CAR and the coding sequence of functional protein are in the same expression frame, which contains a promoter, a nucleic acid sequence encoding the chimeric antigen receptor and a functional protein, and a polyA tailing signal.

In some embodiments, the coding sequence or expression frame is integrated into the genome of CAR-T cells. Therefore, in these embodiments, the genome of CAR-T cells described herein is stably integrated with an expression frame encoding CAR and functional protein described herein.

In some embodiments, the nucleic acid molecule is a nucleic acid construct containing the coding sequences of CAR and/or functional protein described herein, and one or more regulatory sequences operably connected to these sequences. The regulatory sequence can be a suitable promoter sequence. The promoter sequence is usually operably linked to the coding sequence of the protein to be expressed. The promoter may be any nucleotide sequence exhibiting transcriptional activity in the selected host cell, including mutant, truncated and heterozygous promoters, and may be obtained from genes encoding extracellular or intracellular polypeptides homologous or heterologous to the host cell. The regulatory sequence can also be a suitable transcription terminator sequence recognized by the host cell to terminate transcription. The terminator sequence is operably linked to the 3' end of the nucleotide sequence encoding the polypeptide. Any terminator that functions in the selected host cell can be used in this disclosure.

In some embodiments, the nucleic acid construct is a vector. The vector can be a cloning vector, expression vector or homologous recombination vector. Specifically, the coding sequences of CAR and/or functional protein herein can be cloned into many types of vectors, including but not limited to plasmids, phages, phage derivatives, animal viruses and cosmids. The cloning vector can be used to provide the coding sequences of CAR and functional protein of the disclosure, such as a nucleic acid molecule containing the coding sequence of CAR and the coding sequence of functional protein. Expression vectors can be provided to cells in the form of viral vectors. Viruses that can be used as vectors include but are not limited to retroviruses, adenoviruses, adeno-associated viruses, herpesviruses and lentiviruses. The homologous recombinant vector is used to integrate the expression frame described herein into the host genome.

In general, a suitable vector includes a replication starting point, a promoter sequence, a convenient restriction site, and one or more selectable markers that function in at least one organism. For example, when a retroviral vector is used, the retroviral vector usually contains a replication start site, 3'LTR, 5'LTR, the coding sequence of the fusion protein described herein, and an optional selectable marker.

Suitable promoters include but are not limited to immediate early cytomegalovirus (CMV) promoter sequence, extended growth factor-1 (EF-1), simian virus 40 (SV40) early promoter, mouse breast cancer virus (MMTV), human immunodeficiency virus (HIV) long terminal repeat (LTR) promoter, MoMuLV promoter, avian leukemia virus promoter, EB virus immediate early promoter, Rutherford sarcoma virus promoter, and human gene promoter, such as but not limited to actin promoter, myosin promoter, heme promoter and creatine kinase promoter.

Selectable markers include any or both of selectable marker genes or reporter genes to facilitate the identification and selection of expressing cells from cell populations infected with viral vectors. Useful selectable marker genes include, for example, antibiotic resistance genes, such as neo. Suitable reporter genes may include genes encoding luciferase, β-galactosidase, chloramphenicol acetyltransferase, secretory alkaline phosphatase or green fluorescent protein.

The nucleic acid molecules described herein can usually be obtained by PCR amplification. Specifically, primers can be designed according to the nucleotide sequences disclosed herein, and the relevant sequences can be amplified by using a commercially available cDNA library or a cDNA library prepared by a conventional method known to those skilled in the art as a template. When the sequence is long, two or more PCR amplification is often required, and then the amplified fragments are spliced together in the correct order. Alternatively, the nucleic acid molecules described herein may be synthesized directly.

The nucleotide sequence of an exemplary nucleic acid molecule containing the coding sequence of CAR and functional protein can be shown in SEQ ID No: 1. The nucleic acid molecules (especially vectors) described herein can be introduced into host cells by conventional methods, including microinjection, gene gun, electroporation, virus mediated transformation, electron bombardment, calcium phosphate precipitation, etc.

As described herein, the host cell contains the nucleic acid molecules described herein. Host cells include not only T cells ultimately used for disease treatment purposes, but also various cells used in the production of CAR-T cells, such as *E. coli* cells, for providing the coding sequence of the protein of the disclosure or the vector described herein. In some embodiments, a CAR-T cell stably expressing the functional protein described herein is provided herein.

The nucleic acid molecules described herein are also included herein. As described above, the nucleic acid molecules described herein can be prepared by conventional methods in the art. In some embodiments, this disclosure also includes a lentivirus, which includes the expression frame described herein and can integrate the expression frame described herein into the genome of the host cell. The lentivirus described herein can be prepared by a method well known in the art. For example, a lentivirus vector containing the expression frame described herein is prepared firstly, then the virus is packaged in a suitable host cell, and the required lentivirus is isolated and purified. The reagents used for lentivirus packaging are well known in the art. For example, the conventional lentivirus vector system (Tronolab) includes pRsv-REV, pMDlg-pRRE, pMD2G and target interference plasmid.

This paper also includes a CAR-T cell culture containing the CAR-T cells described herein and a suitable medium. The culture medium may be a culture medium conventionally used in the art for culturing CAR-T cells.

The disclosure also provides a pharmaceutical composition containing CAR-T cells and pharmaceutically acceptable excipients. In this disclosure, pharmaceutically acceptable excipients refer to carriers and/or excipients that are pharmacologically and/or physiologically compatible with subjects and active ingredients, including but not limited to pH regulators, surfactants, adjuvants and ionic strength enhancers. More specifically, suitable pharmaceutically acceptable excipients can be excipients commonly used in CAR-T cell administration in the art.

Generally, the pharmaceutical composition contains a therapeutically effective amount of CAR-T cells. Therapeutic effective dose refers to the dose that can treat, prevent, reduce and/or alleviate diseases or conditions in a subject. The effective amount of treatment can be determined according to the patient's age, gender, disease and its severity, other physical conditions and other factors. In this disclosure, subjects or patients usually refer to mammals, especially humans.

In this disclosure, diseases suitable for treatment using the nucleic acid molecules, CAR-T cells and pharmaceutical compositions described herein are related to single chain antibodies in the chimeric antigen receptors expressed by the nucleic acid molecules and CAR-T cells. Therefore, the diseases described herein include various types of cancer related to tumor antigens mentioned above, including solid tumors and hematologic tumors, such as adenocarcinoma, lung cancer, colon cancer, colorectal cancer, breast cancer, ovarian cancer, cervical cancer, gastric cancer, cholangiocarcinoma, gallbladder cancer, esophageal cancer, pancreatic cancer and prostate cancer, and other solid tumors, and leukemia and lymphoma, such as B cell lymphoma, mantle cell lymphoma, acute lymphocytic leukemia, chronic lymphocytic leukemia, hairy cell leukemia and acute myeloid leukemia, etc.

In some embodiments, a kit containing the vector described herein is also provided. The kit may also contain various reagents suitable for transfecting the vector into cells and optional instructions for guiding those skilled in the art to transfect the recombinant expression vector into cells.

The disclosure also provides a method of treatment of a disease, which comprises administrating a therapeutic effective amount of engineered T cells according to any embodiment of this disclosure, preferably engineered CAR-T cells according to any embodiment of this disclosure to a subject in need thereof. According to the existing cellular immunotherapy, the drug can be administered in an appropriate way (such as intravenous infusion) according to the patient's disease, severity, age, gender and other factors. Preferably, the disease is the disease described in any embodiment herein. In some embodiments, the HLA-E genotype of the patient's T cells is 01:03/01:03, and the T cells, preferably CAR-T cells containing one or more of protein US11 from HCMV, protein k5 from KSHV, protein US6 from HCMV, protein UL49.5 from BHV-1 and protein UL49.5 from EHV-1 and/or containing the coding sequence of one or more of these proteins are administrated to the patient. In some embodiments, the HLA-E genotype of the patient's T cells is 01:01/01:01, and T cells, preferably CAR-T cells containing protein US11 from HCMV and/or protein k5 from KSHV and/or containing coding sequences of one or both of these proteins are administrated to the patient.

The disclosure comprises the following embodiments:
Item 1. A CAR-T cell, wherein the CAR-T cell expresses a chimeric antigen receptor and a functional protein capable of down regulating the expression of HLA class I molecules on the cell surface; preferably, the expression level of HLA class I molecules on the surface of the CAR-T cells expressing the chimeric antigen receptor and the functional protein is less than 50% of that of the control CAR-T cells expressing the same chimeric antigen receptor but not the functional protein.
Item 2. The CAR-T cell according to Item 1, wherein the CAR-T cell contains a coding sequence of the chimeric antigen receptor and a coding sequence of the functional protein; preferably, the CAR-T cell contains an expression frame of the chimeric antigen receptor and an expression frame of the functional protein, or the coding sequence of the chimeric antigen receptor and the coding sequence of the functional protein are in the same expression frame.
Item 3. CAR-T cells according to Item 1 or 2, wherein,
   The chimeric antigen receptor specifically binds one or more of the following tumor antigens selected from the group consisting of EGFRvIII, mesothelin, gD2, Tn antigen, sTn antigen, Tn-O-glycopeptide, sTn-O-glycopeptide, PSMA, CD97, TAG72, CD44v6, CEA, EpCAM, KIT, IL-13RA2, Leguman, GD3, CD171, IL-11RA, PSCA, MAD-CT-1, MAD-CT-2, VEGFR2, LewisY, CD24, PDGFR-β, SSEA-4, folate receptor α, ERBB, HER2/neu, MUC1, EGFR, NCAM, Ephrin B2, CAIX, LMP2, SLE, HMWMAA, o-acetyl-GD2, folate receptor β. TEM1/CD248, TEM7R, FAP, Legumain, HPV E6 or E7, ML-IAP, CLDN6, TSHR, GPRC5d, ALK, polysialic acid, FOS related antigen, neutrophil elastase, TRP-2, CYP1B1, sperm protein 17, β Human chorionic gonadotropin, AFP, thyroglobulin, PLAC1, globoH, RAGE1, MN-CA IX, human telomerase reverse transcriptase, intestinal carboxylesterase, mut HSP 70-2, NA-17, NY-BR-1, UPK2, HAVCR1, ADRB3, PANX3, NY-ESO-1, GPR20, Ly6k, OR51E2, TARP, GFRα4 and the polypeptide fragments of any of these antigens presented on MHC, as well as CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79A, CD79B, CD80, CD81, CD82, CD83, CD84, CD85, CD86, CD123, CD135, CD138, CD179, CD269, Flt3, ROR1, BCMA, FcRn5, FcRn2, CS-1, CXCR4, CXCR5, CXCR7, IL-7/3R, IL-7/4/3R, and IL4R; or
   The functional proteins capable of down regulating the expression of HLA class I molecules on the cell surface are selected from HSV, BHV-1, EHV-1/4, PRV, HSV-1/2, VZV, EBV, HCMV, MCMV, RhCMV, HHV-6/7, KSHV, MHV-68, the functional proteins that can directly target degrade HLA class I in cowpox virus and adenovirus, functional proteins that can down regulate the expression of HLA class I molecules through TAP proteins and functional proteins that can down regulate the expression of HLA class I molecules through lysosomes; preferably, the functional protein is selected from proteins US 11 and US6 from HCMV, protein UL49.5 from BHV-1, protein UL49.5 from EHV-1 and protein k5 from KSHV.
Item 4. A nucleic acid molecule, wherein the nucleic acid molecule is selected from:
   (1) A nucleic acid molecule containing a coding sequence of chimeric antigen receptor and a coding sequence of a functional protein capable of down regulating the expression of HLA class I molecules on the cell surface; and
   (2) A complementary sequence of the nucleic acid molecule of (1);
   Preferably, the chimeric antigen receptor and the functional protein are as described in Item 3.
Item 5. A nucleic acid construct, wherein the nucleic acid construct contains the nucleic acid molecule described in Item 4.
Item 6. The nucleic acid construct according to Item 5, wherein,
   The nucleic acid construct comprises an expression frame of the chimeric antigen receptor and an expression frame of the functional protein; or the nucleic acid construct is an expression frame, wherein the coding sequence of the chimeric antigen receptor and the coding sequence of the functional protein are in the expression frame; or
   The nucleic acid construct is a cloning vector or an expression vector.
Item 7. A lentivirus containing the nucleic acid construct according to Item 5 or 6.
Item 8. A host cell containing the nucleic acid molecule according to Item 4 or the nucleic acid construct according to Item 5 or 6 or the lentivirus according to Item 7.
Item 9. A pharmaceutical composition, wherein the pharmaceutical composition contains CAR-T cells according to any one of Items 1-3.
Item 10. Use of the functional protein or its coding sequence according to Item 3 in the preparation of CAR-T cells with down regulated expression of HLA class I molecules on the cell surface, or in the preparation of CAR-T cells for cancer treatment.

The embodiment of the disclosure will be described in detail below in combination with an example. Those skilled in the art will understand that the following examples are only used to illustrate the disclosure and should not be regarded as limiting the scope of the disclosure. If the specific technology or conditions are not indicated in the example, it shall be carried out in accordance with the technology or conditions described in the literature in the field (e.g., J. Sambrook et al., Molecular Cloning: A Laboratory Manual, 3rd ed., translated by Huang Peitang et al., Science Press) or in accordance with the product instruction. The reagents or instruments used without the manufacturer indicated are conventional products that can be commercially obtained.

### 1. Experimental materials and results

### (1) Instruments and materials:

Instruments: biosafety cabinet (Haier, HR40-IIA2), CO2 incubator (Thermo, 3111), Flow cytometry (BD, FACSCantoll), plate reader (Molecular Derices, SpectraMax M4)

Reagents: anti human Anti-HLA class I antibody (APC) (Biolegend, 311410), anti human HLA-A antibody (Bio-Techne, NBP2-45320), anti human HLA-B antibody (Bio-Techne, NBP2-45000), anti human HLA-E antibody (Biolegend, 342612), anti-CD3 antibody (BV421) (Biolegend, 300434), anti-TCR antibody (PE-Cy7) (Biolegend, 306720), FBS (Lonsera, S711-001S), X-vivo 15 (Lonza, 04-418q), Dynabeads CD3/CD28 (Lifetechnology, 40203d), Ficoll (Dayou, DKW-LSH-0250), Tscm (Novoprotein, GMP-1647), Novonectin (Novoprotein, GMP-CH38), anti-human CCR7 (BV421) (BD, 562555), anti-human CD45RA (PE-Cy7) (BD, 560675), Luciferase test (Promega, E6120), anti-human CD3 (FITC) (BD, 562555), anti-human CD4 (BV510) (BD, 563094), anti-human CD8 (APC-Cy7) (BD, 557834), anti-human CD25 (PE) (BD, 555432), anti-human CD69 (APC) (BD, 553237), CAR19 specific monoclonal antibody (self-standard), INF-γ ELISA Kit (R & D, DIF50).

### (2) Test method

### I. Experimental method

### 1) Vector construction

Human CD8α hinge region, human CD8 transmembrane region, 41BB intracellular region, human CD3, and EHV1 UL49.5 gene sequence information were searched and obtained from NCBI website database, and the clone number of anti-CD19 scFv is FMC63. These sequences are codon optimized on the website of https://www.thermofisher.com/order/geneartgenes to ensure that they are more suitable for human cell expression without changing the coded amino acid sequence.

Overlapping PCR was used to connect the above sequences according to the order of anti-CD19 scFv gene, human CD8 hinge region gene, human CD8 transmembrane region gene, 41BB intracellular region gene, human CD3 intracellular region, F2A and EHV1 UL49.5 gene sequence to form complete CD19-CAR-F2A-EHV1 UL49.5 gene sequence information (the sequence containing the coding sequence of signal peptide is shown in SEQ ID No: 1, and the amino acid sequence is shown in SEQ ID No: 2).

The nucleotide sequence of the CAR molecule was seamlessly cloned into Bamh1-Ecor1 site of lentivirus plasmid pWPXL (Addgene) and transformed into competent *E. coli* (DH5α, Beijing bomaide Biotechnology Co., Ltd.).

The recombinant plasmid was sent to Suzhou Jinweizhi Biotechnology Co., Ltd. for sequencing. The sequencing results were compared with the sequence of the fitted CD19-CAR-F2A-EHV1 UL49.5 to verify whether the sequence was correct. The sequencing primer was TCAAGCCTCAGACAGTGGTTC (SEQ ID No: 3).

After confirmation by sequencing, the plasmid was extracted and purified using the plasmid purification kit of Qiagen. The purified plasmid was transfected into 293T cells by calcium phosphate method for lentivirus packaging experiment (Molecular Therapy-Methods & Clinical Development, 2016, 3: 16017). The prepared lentivirus was named PCTL206.

Lentiviruses PCTL135 (CAR19-F2A-GFP, control), PCTL199 (CAR19-F2A-HCMV US2), PCTL200 (CAR19-F2A-HCMV US11), PCTL201 (CAR19-F2A-HCMV US6), PCTL202 (CAR19-F2A-HSV-1 ICP47), PCTL203 (CAR19-F2A-5 E3-19K), PCTL204 (CAR19-F2A-RHCMV Rh178), PCTL205 (CAR19-F2A-BHV-1 UL49.5), PCTL207 (CAR19-F2A-EBV BNLF2a), PCTL208 (CAR19-F2A-CPXV012), PCTL209 (CAR19-F2A-HCMV US3), PCTL210 (CAR19-F2A-MHV68 mK3), PCTL211 (CAR19-F2A- CPXV203), PCTL212 (CAR19-F2A-KSHV k3), PCTL213 (CAR19-F2A-KSHV k5) were prepared using the same method with different viral proteins to replace that in PCTL206, wherein the amino acid sequences of viral proteins HCMV US2, HSV-1 ICP47, human adenovirus 5 E3-19K, RhCMV Rh178, EBV BNLF2a, cowpox virus CPXV012, HCMV US3, MHV68 mK3, cowpox virus CPXV203, KSHV k3, HCMV US 11, HCMV US6, BHV-1 UL49.5 and KSHV k5 are respectively shown in the amino acid sequences shown in the following UniprotKB accession numbers: C8CFI0, P03170, Q8BEL5, Q7TFG4, POC739, U5TIW7, Q910T7, 041933, GOXWR7, A0A386AVI8, P09727, P14334, Q77CE4 and F5H9K4. More specifically, the coding sequence of HCMV US 11 is shown as 1546-2193 bases of SEQ ID No: 4; the coding sequence of HCMV US6 is shown as 1546-2097 bases of SEQ ID No: 5; the coding sequence of BHV-1 UL49.5 is shown as 1546-2097 bases of SEQ ID No: 6; the coding sequence of KSHV k5 is shown as 1546-2316 bases of SEQ ID No: 7.

### 2) Isolation of PBMC from peripheral blood, isolation and activation of T cells, lentivirus transduction and in vitro culture

Healthy donors with negative HBV, HCV and HIV tests were selected. 100ml blood was drawn from the median vein of elbow, and the buffy coat of PBMC was separated by Ficoll density gradient centrifugation. The percentage of CD3+ T cells was detected by whole blood flow cytometry, and the number of CD3+ T cells was calculated. According to the ratio of Dynabeans CD3/CD28 to CD3+ T cells of 3:1, the use amount of magnetic beads was absorbed, incubated with buffy coat cells for 30min, and CD3+ T cells were isolated, CD3 + T cells were activated by Dynabeads CD3/CD28 (Lifetechnology, 40203D) for 24 hours, and the proportion of CD25+CD69+ T cells was detected by flow cytometry. After CD3+ T activation, lentivirus transduction was conducted. The 24 well plate coated with Novonectin was incubated at 37°C for 2 hours. The cell suspension and various lentiviruses (MOI = 8), F108 (10ug/ml) and Tscm (2U/ml) prepared above were configured into a transduction system placed in the coated 24 well plate, the cell density was adjusted to 1.0E + 06/ml, centrifuged at 500g for 30 minutes, and then cultured in a 37 °C CO₂ incubator for 48 hours. After transfection, the cells were cultured in Xvivo15 medium containing 5% FBS, supplemented with TSCM (final concentration of 2U/ml) every other day, counted, adjusted to the cell density of 0.5E + 06/ml, and cultured to harvest the cells on Day 8-10.

### 3) Target cells stimulated CAR-T cells in each group

CAR-T cells in each group were cultured in vitro for 8-10 days, 1.0E + 07 cells were counted, the cell density was adjusted to 1.0E + 06/ml, cultured with Xvivo15 (excluding TSCM), and the target cell K562-CD19 was added according to the effect target ratio of 10:1 to stimulate CAR-T cells. The CAR positive rate of cells in each group and the average fluorescence intensity of HLA class I of Car positive cells were detected by flow cytometry.

### 4) The CAR positive rate of CAR-T cells and the average fluorescence intensity of HLA class I of CAR+ T cells in each group were detected by flow cytometry

CAR-T cells in each group were counted, 5.0E + 05 cells were taken into different 1.5ml EP tubes, centrifuged at 2000rpm for 5min, the cells were collected, the culture medium was discarded, the cells were resuspended and washed twice with sterile 4% BSA, and then the cells were resuspended with 100ul 4% BSA. 8ul of anti-human HLA class I antibody (APC) (Biolegend, Cat #: 311410) antibody was added to each tube, vortex mixed evenly, and incubated at 4 °C for 30min; after dyeing, the cells were washed repeatedly, CAR19 specific antibody was diluted at 1:500, the cells were resuspended with diluted antibody solution, 200ul per tube, vortex mixed evenly, incubated at 4 °C for 30min. After dyeing, the cells were washed repeatedly, resuspended with 500ul 4% BSA, with 4ul of 7AAD antibody added to each tube, vortex mixed evenly, incubated at room temperature in dark for 10min. After incubation, the cells were transferred to flow tube, and tested on the machine.

### 5) Cell phenotype analysis

CAR-T cells in each group were counted, and 1.0E + 06 cells were taken into different 1.5ml EP tubes, centrifuged at 2000rpm for 5min, the cells were collected, the culture medium was discarded, the cells were resuspended and washed twice with sterile 4% BSA, and then the cells were resuspended with 200ul 4% BSA. 5ul of anti-human CD45RA (PE-Cy7) and anti-human CCR7 (BV421) antibodies were added to each sample tube, mixed with a vortex mixer, and incubated at 4 °C for 30min. After the staining was completed, the cells were washed repeatedly, resuspended with 500ul 4% BSA, with 4ul of 7AAD antibody added to each tube, vortex mixed evenly, incubated at room temperature in the dark for 10min, transferred to the flow tube after incubation, and tested on the machine.

### 6) Cell killing

NC-T (T cells not transfected with lentivirus) and CAR-T cells in each group were taken, observed under the microscope whether the cell growth state was normal, blown and mixed evenly, and NC-T and CAR-T cells in each group were collected in a centrifuge tube, counted, collected by centrifugation, and the cell precipitation collected by centrifugation was resuspended with T cell culture medium X-VIVO 15 (excluding Tscm), the cell density was adjusted to 5.0E + 07 cells/ml; the target cells were taken, observed whether under the microscope the cell state was normal, the target cells were collected in 15ml or 50ml centrifuge tubes respectively, counted, and the cell precipitation collected by centrifugation was resuspended with RPMI 1640 (excluding FBS), and the cell density was adjusted to 5.0E + 06 cells/ml; the effector cells NC-T and CAR-T with adjusted density were mixed with the target cells according to different effect target ratios (1:1, 2.5:1, 5:1, 10:1 and 20:1) in a 1.5ml centrifuge tube, the total volume was made up by 200 with x-vivo 15 (the target cells are 10ul, and the amount of effector cells was determined according to the effect target ratio). The 200 prepared killing system was moved into 96 well V-shaped plates for 24 hours, the cells in 96 well V-plate were gently blown and mixed, and 100 cells µ0 cell suspension was respectively transferred into 96 well plate with impermeable white wall bottom, and 80 µL ONE-Glo^{™} Luciferase Assay Substrate was added, mixed evenly by blowing and suction, incubated in the dark at room temperature for 10 minutes, and then the fluorescence intensity was tested with Luminoskan Ascent chemiluminescence analyzer.

### II. Experimental results

### 1. Functional proteins capable of down regulating the expression of HLA class I molecules on the surface of CAR-T cells were screened.

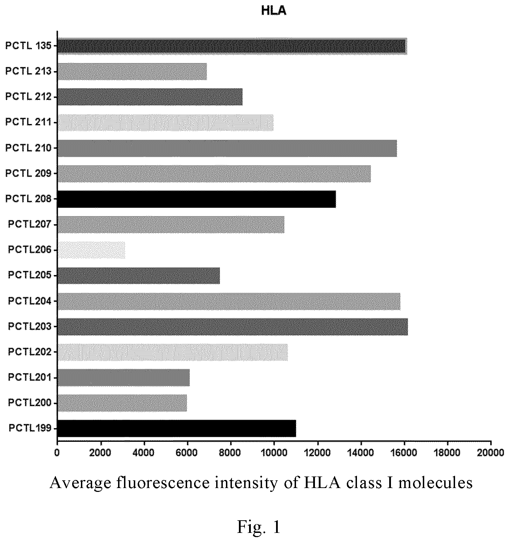

Fourteen viral proteins with down-regulated cell surface function were synthesized and constructed into CAR19 lentivirus. Each group of lentivirus was transfected into T cells, and T cells were cultured *in vitro* until Day 8. The transfection efficiency of CAR19 and the average fluorescence intensity of HLA class I of CAR19 + T cell population were detected by flow cytometry (Fig. 1, Table 1).

**Table 1: Average fluorescence intensity of HLA class I of T cells in each group**

| Viral vector | CAR+% | Average fluorescence intensity of HLA class I | Down regulated to (sample / control) | Viral vector | CAR+% | Average fluorescence intensity of HLA class I | Down regulated to (sample/control) |
|---|---|---|---|---|---|---|---|
| PCTL199 | 54.6 | 10997 | 68% | PCTL207 | 44.2 | 10478 | 65% |
| PCTL200 | 51.1 | 5975 | 37% | PCTL208 | 58.7 | 12826 | 80% |
| PCTL201 | 54.8 | 6098 | 38% | PCTL209 | 45.6 | 14454 | 90% |
| PCTL202 | 57.8 | 10616 | 66% | PCTL210 | 53.3 | 15649 | 97% |
| PCTL203 | 63.4 | 16150 | 100% | PCTL211 | 41.7 | 9971 | 62% |
| PCTL204 | 49.7 | 15797 | 98% | PCTL212 | 36 | 8534 | 53% |
| PCTL205 | 48.5 | 7495 | 46% | PCTL213 | 68 | 6888 | 43% |
| PCTL206 | 44.8 | 3118 | 19% | PCTL135 | 30.5 | 16133 | 100% |

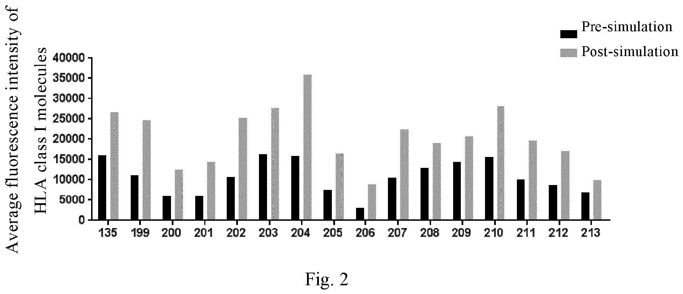

On Day 9 of *in vitro* culture, T cells in each group were stimulated with target cell K562-CD19, the effect target ratio was 10:1, and stimulated repeatedly for 2 days. The average fluorescence intensity of HLA class I of T cell CAR19⁺ cell population in each group was detected by flow cytometry. The results were shown in Fig. 2. The results showed that PCTL200, PCTL201, PCTL205, PCTL206 and PCTL213 lentiviruses had strong ability to prepare CAR-T cells with down-regulated expression of HLA class I molecules.

### 2. Functional determination of PCTL200, PCTL201, PCTL205, PCTL206 and PCTL213 molecules

### 1) PBMCs isolated from peripheral blood and T cell activation

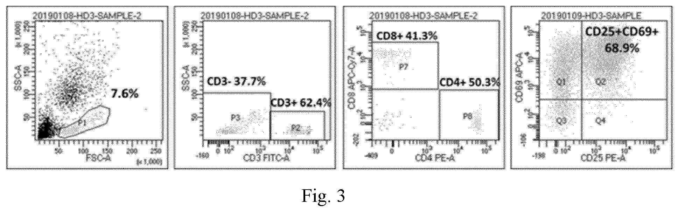

1.24E + 08 PBMCs were isolated from 100ml peripheral blood, and the proportions of CD3+ T, CD4+ T and CD8+ T in peripheral blood were detected by flow cytometry. Among them, CD3+ T accounted for 62.4% of buffy coat (monocytes and lymphocytes), CD4+ T/CD8+ T was 1.2, and dynabeans CD3/CD28 sorted and activated CD3+ T, and the activation efficiency was 68.9% after 24h (Fig. 3).

### 2) T cell transfection efficiency, average fluorescence intensity of HLA class I, in vitro proliferation multiple, cytotoxicity test, T cell phenotype analysis

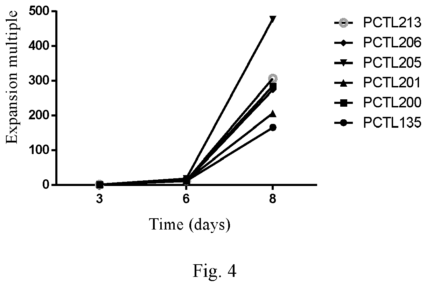

T cells were transfected with lentivirus in each group, T cells were counted every other day, T cells were cultured in vitro until Day 8, the transfection efficiency of CAR19 was detected by flow cytometry, and the cell proliferation multiple (the number of cells on Day 8/the number of cells used for transfection) was calculated. The results are shown in Table 2 and Fig. 4 below, wherein the CAR19 positive rate of PCTL135 group was 65.6%, that in PCTL200 group was 37.4%, that in PCTL201 group was 46%, that in PCTL205 group was 55.8%, that in PCTL206 group was 54.6%, and that in PCTL213 group was 55.7%.

**Table 2: transfection efficiency and proliferation multiple of T cells in each group**

| Group | Viral vector | Titer | Transfection efficiency (Day 8) | Proliferation multiple |
|---|---|---|---|---|
| 1 | PCTL135 | 1.36E+07 | 65.6% | 165.60 |
| 2 | PCTL200 | 7.20E+07 | 37.4% | 284.40 |
| 3 | PCTL201 | 1.40E+08 | 46% | 206.15 |
| 4 | PCTL205 | 1.32E+08 | 55.8% | 477.00 |
| 5 | PCTL206 | 1.22E+08 | 54.6% | 274.00 |
| 6 | PCTL213 | 1.04E+08 | 55.7% | 306.25 |

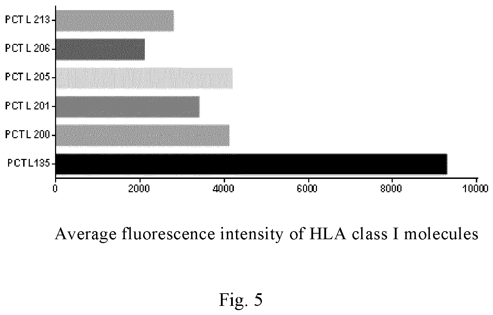

The average fluorescence intensity of HLA class I in CAR19 + T cell population was analyzed by flow cytometry. Results as shown in Table 3 and Fig. 5, the average fluorescence intensity of HLA class I of T cells in each experimental group was significantly lower than that of PCTL135 in the control group, indicating that the five viral proteins tested can down regulate the expression of HLA class I molecules on the surface of T cells, and allogeneic CAR-T can be prepared by expressing related viral proteins to target HLA class I molecules.

**Table 3: Average fluorescence intensity of HLA class I of T cells in each group**

| Viral vector | Transduction efficiency% | Average fluorescence intensity of HLA class I | Down regulated to (sample/control) |
|---|---|---|---|
| PCTL135 | 65.6% | 9273 | 100% |
| PCTL200 | 37.4% | 4100 | 44% |
| PCTL201 | 46% | 3382 | 36% |
| PCTL205 | 55.8% | 4179 | 45% |
| PCTL206 | 54.6% | 2090 | 23% |
| PCTL213 | 55.7% | 2776 | 30% |

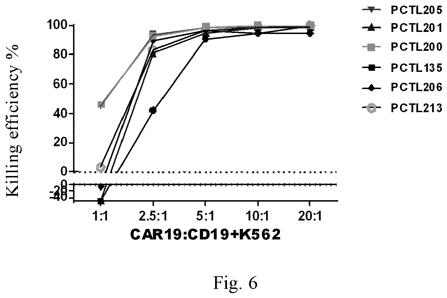

T cells in each group were cultured *in vitro* until Day 8, and co cultured with the target cell line k562-CD19-luc for 24h according to the effect target ratio of 20:1, 10:1, 5:1, 2.5:1 and 1:1 respectively. The cytotoxicity of T cells in each group was detected by luciferase reporter gene detection system. The results are shown in Table 4 and Fig. 6.

**Table 4: in vitro killing efficiency of T cells in each group**

| Effect target ratio | PCTL135 | PCTL200 | PCTL201 | PCTL205 | PCTL206 | PCTL213 |
|---|---|---|---|---|---|---|
| 1:1 | -247% | 46% | 68% | 45% | -6% | 3% |
| 2.5:1 | -42% | 93% | 81% | 94% | 90% | 83% |
| 5:1 | 91% | 99% | 95% | 99% | 97% | 97% |
| 10:1 | 95% | 100% | 99% | 99% | 95% | 99% |
| 20:1 | 95% | 100% | 99% | 100% | 100% | 100% |

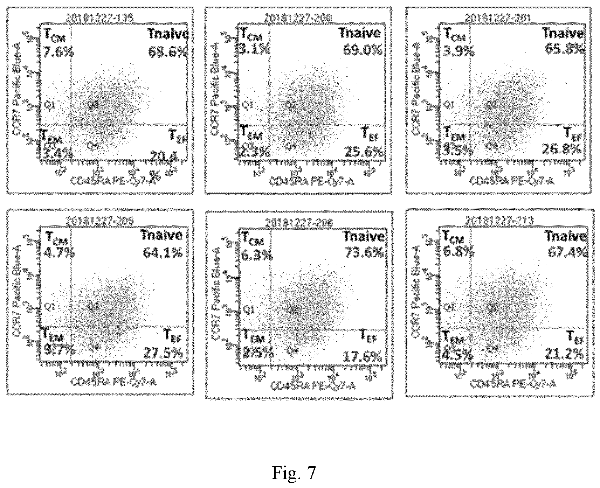

The T cells of each group were cultured in vitro until Day 8. The expression of CCR7 and CD45RA of T cells in each group was detected by flow cytometry, and the T cell phenotype was analyzed. There was no significant difference among the cell phenotypes of each group, and about 60% of the cells were in T-naive differentiation stage (Fig. 7).

### 3) The average fluorescence intensity of HLA class I of T cells in each group after stimulation by target cells

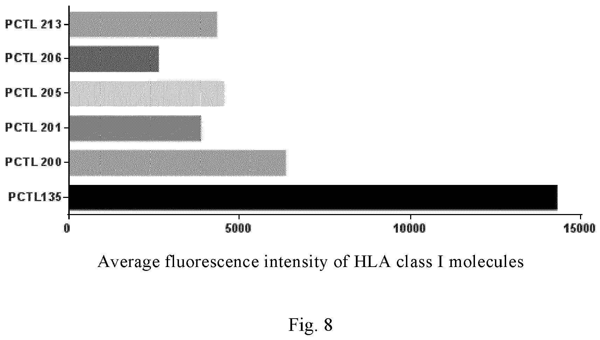

On Day 9 of *in vitro* culture, the target cell K562-CD19 was used to stimulate T cells in each group for 2 days, with an effect target ratio of 10:1. The average fluorescence intensity of HLA class I of CAR19⁺ cell population of T cells in each group was detected by flow cytometry. The results are shown in Table 5 and Fig. 8. The results show that the average fluorescence intensity of HLA class I in each group is significantly lower than that in the control group, especially in PCTL206 group.

**Table 5: average fluorescence intensity of HLA class I of T cells in each group after stimulation by target cells**

| Viral vector | Killing efficiency% (E: T = 10:1) | Average fluorescence intensity of HLA class I molecules |
|---|---|---|
| PCTL135 | 69.5 | 14259 |
| PCTL200 | 52.9 | 6328 |
| PCTL201 | 61.8 | 3839 |
| PCTL205 | 69.8 | 4525 |
| PCTL206 | 68.7 | 2598 |
| PCTL213 | 71.8 | 4300 |

### 3. Regulation of the expression of different subtypes of HLA class I by PCTL200 and PCTL213

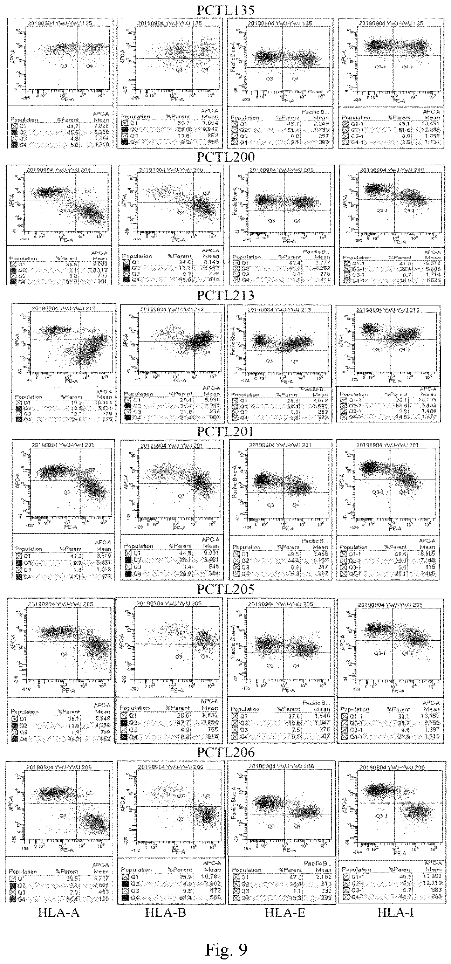

Fig. 9 and Tables 6-7 show the average fluorescence intensities of different HLA class I molecules (HLA-A, HLA-B and HLA-E) in each group in CAR19⁺ T cell population and their ratio to the control. The results of PCTL135 group (control), PCTL200 group and PCTL213 group showed that compared with the control, PCTL200 group and PCTL213 group significantly down regulated HLA-A and HLA-B molecules on the cell surface, but there was no significant difference with the control for HLA-E molecules.

**Table 6: average fluorescence intensity of HLA of T cells in each group**

| virus | Average fluorescence intensity of HLA-A molecule | Average fluorescence intensity of HLA-B molecule | Average fluorescence intensity of HLA-E molecule | Average fluorescence intensity of HLA class I molecules |
|---|---|---|---|---|
| PCTL135 | 7658 | 8367 | 1678 | 11800 |
| PCTL200 | 443 | 929 | 1822 | 4253 |
| PCTL201 | 1385 | 2089 | 1023 | 4761 |
| PCTL205 | 1680 | 3023 | 915 | 4846 |
| PCTL206 | 449 | 728 | 660 | 2132 |
| PCTL213 | 1098 | 2389 | 1559 | 5437 |

**Table 7: ratio of average fluorescence intensity of HLA of T cells in each group to the control**

| virus | HLA-A molecules down regulated to (sample/control ) | HLA-B molecule down regulated to (sample/control ) | HLA-E molecule down regulated to (sample/control ) | HLA class I molecules down regulated to (sample/control ) |
|---|---|---|---|---|
| PCTL13 5 | 100% | 100% | 100% | 100% |
| PCTL20 0 | 6% | 11% | **100%** | 36% |
| PCTL20 1 | 18% | 25% | **61%** | 40% |
| PCTL20 5 | 22% | 36% | **55%** | 41% |
| PCTL20 6 | 6% | 9% | **39%** | 18% |
| PCTL21 3 | 14% | 29% | **93%** | 46% |

### 4. Regulation of expression of different HLA class I subtypes in T cells with different HLA-E genotypes

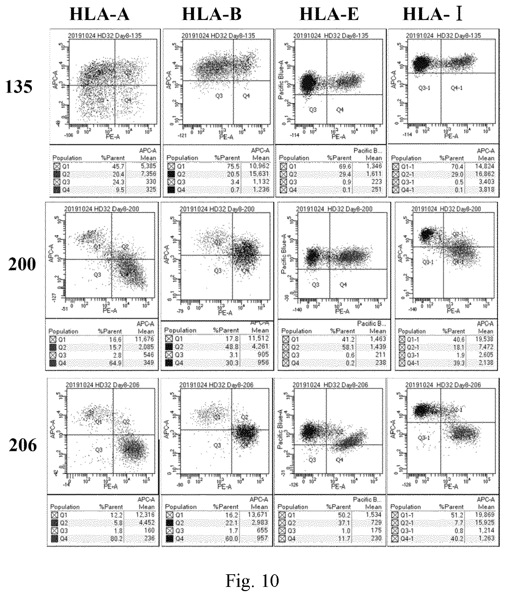

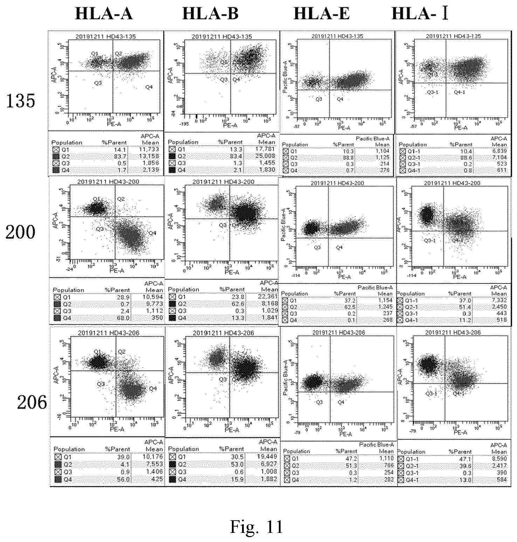

Figs. 9-11 and Tables 8-11 show the average fluorescence of various HLA class I molecules (HLA-A, HLA-B and HLA-E) in CAR19⁺ T cell populations of different HLA-E genotypes and their ratios to control. It can be seen that PCTL201, PCTL205 and PCTL206 groups have selectivity for T cells of different HLA-E genotypes: for CAR19⁺ T cell population with HLA-E genotype of homozygous 01:03/01:03, PCTL206 group down regulates HLA-A and HLA-B molecules on the cell surface, but there is no significant difference with the control for HLA-E molecules (Fig. 11); for CAR19 + T cell population with HLA-E genotype of homozygous 01:01/01:01, PCTL206 group down regulated HLA-A, HLA-B and HLA-E molecules on the cell surface (Fig. 10).

**Table 8: average fluorescence intensity of HLA of T cells in each group (Fig. 10)**

| virus | Average fluorescence intensity of HLA-A molecule | Average fluorescence intensity of HLA-B molecule | Average fluorescence intensity of HLA-E molecule | Average fluorescence intensity of HLA class I molecules |
|---|---|---|---|---|
| PCTL135 | 5122 | 15156 | 1606 | 16817 |
| PCTL200 | 687 | 2995 | 1435 | 3820 |
| PCTL206 | 520 | 1502 | 609 | 3620 |

**Table 9: ratio of average fluorescence intensity of HLA of T cells in each group to the control (Fig. 10)**

| virus | HLA-A molecules down regulated to (sample/control ) | HLA-B molecule down regulated to (sample/control ) | HLA-E molecule down regulated to (sample/control ) | HLA class I molecules down regulated to (sample/control ) |
|---|---|---|---|---|
| PCTL13 5 | 100% | 100% | 100% | 100% |
| PCTL20 0 | 13% | 20% | 89% | 23% |
| PCTL20 6 | 10% | 10% | 38% | 22% |

**Table 10: average fluorescence intensity of HLA of T cells in each group (Fig. 11)**

| virus | Average fluorescence intensity of HLA-A molecule | Average fluorescence intensity of HLA-B molecule | Average fluorescence intensity of HLA-E molecule | Average fluorescence intensity of HLA class I molecules |
|---|---|---|---|---|
| PCTL135 | 12939 | 24439 | 1118 | 7046 |
| PCTL200 | 446 | 7059 | 1243 | 2104 |
| PCTL206 | 911 | 5763 | 755 | 1964 |

**Table 11: ratio of average fluorescence intensity of HLA of T cells in each group to the control (Fig. 11)**

| virus | HLA-A molecules down regulated to (sample/control ) | HLA-B molecule down regulated to (sample/control ) | HLA-E molecule down regulated to (sample/control ) | HLA class I molecules down regulated to (sample/control ) |
|---|---|---|---|---|
| PCTL13 5 | 100% | 100% | 100% | 100% |
| PCTL20 0 | 3% | 29% | 100% | 30% |
| PCTL20 6 | 7% | 24% | 68% | 28% |

## Claims

1. An engineered T cell, wherein the engineered T cell expresses a functional protein that can differentially regulate the expression of HLA class I molecules on the cell surface, wherein the expression levels of HLA-A and HLA-B on the cell surface of the engineered T cell are 50% or less, preferably 5-50% of the T cells that do not express the functional protein, wherein the expression level of HLA-E on the cell surface is more than 50%, preferably 60-100% of the T cells that do not express the functional protein.

2. The engineered T cell according to claim 1, wherein the engineered T cell further express chimeric antigen receptor, so that the engineered T cell is a CAR-T cell, wherein the CAR-T cell contains a coding sequence of the chimeric antigen receptor and a coding sequence of the functional protein; preferably, the CAR-T cell contains an expression frame of the chimeric antigen receptor and an expression frame of the functional protein, or the coding sequence of the chimeric antigen receptor and the coding sequence of the functional protein are in the same expression frame.

3. The engineered T cell according to claim 2, wherein,
the chimeric antigen receptor specifically binds one or more of tumor antigens selected from the group consisting of: EGFRvIII, mesothelin, gD2, Tn antigen, sTn antigen, Tn-O-glycopeptide, sTn-O-glycopeptide, PSMA, CD97, TAG72, CD44v6, CEA, EpCAM, KIT, IL-13RA2, Leguman, GD3, CD171, IL-11RA, PSCA, MAD-CT-1, MAD-CT-2, VEGFR2, LewisY, CD24, PDGFR-β, SSEA-4, folate receptor α, ERBB, HER2/neu, MUC1, EGFR, NCAM, Ephrin B2, CAIX, LMP2, SLE, HMWMAA, o-acetyl-GD2, folate receptor β, TEM1/CD248, TEM7R, FAP, Legumain, HPV E6 or E7, ML-IAP, CLDN6, TSHR, GPRC5d, ALK, polysialic acid, FOS related antigen, neutrophil elastase, TRP-2, CYP1B1, sperm protein 17, β Human chorionic gonadotropin, AFP, thyroglobulin, PLAC1, globoH, RAGE1, MN-CA IX, human telomerase reverse transcriptase, intestinal carboxylesterase, mut HSP 70-2, NA-17, NY-BR-1, UPK2, HAVCR1, ADRB3, PANX3, NY-ESO-1, GPR20, Ly6k, OR51E2, TARP, GFRα4 and the polypeptide fragments of any of these antigens presented on MHC, as well as CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79A, CD79B, CD80, CD81, CD82, CD83, CD84, CD85, CD86, CD123, CD135, CD138, CD179, CD269, Flt3, ROR1, BCMA, FcRn5, FcRn2, CS-1, CXCR4, CXCR5, CXCR7, IL-7/3R, IL-7/4/3R, and IL4R.

4. The engineered T cell according to any of claims 1-3, wherein,
the functional proteins capable of regulating the expression of HLA class I molecules on the cell surface are selected from the group consisting of: HLA class I in HSV, BHV-1, EHV-1/4, PRV, HSV-1/2, VZV, EBV, HCMV, MCMV, RhCMV, HHV-6/7, KSHV, MHV-68, functional proteins that can directly target degrade HLA class I in cowpox virus and adenovirus, functional proteins that can down regulate the expression of HLA class I molecules through TAP protein and functional proteins that can down regulate the expression of HLA class I molecules through lysosomes; preferably, the functional protein is selected from the group consisting of: proteins US11 and US6 from HCMV, protein UL49.5 from BHV-1, protein UL49.5 from EHV-1 and protein k5 from KSHV.

5. The engineered T cell according to any of claims 1-3, wherein
the functional protein is selected from protein US 11 from HCMV and protein k5 from KSHV, and the expression of HLA-A and HLA-B and the expression of HLA-E are differentially regulated on the cell surface of the engineered T cell, or
the functional protein is selected from protein US6 from HCMV, protein UL49.5 from BHV-1 and protein UL49.5 from EHV-1, the genotype of HLA-E of the engineered T cell is 01:03/01:03, and the expression of HLA-A and HLA-B and the expression of HLA-E are differentially regulated on the cell surface of the engineered T cell, or
the functional protein is selected from protein US6 from HCMV, protein UL49.5 from BHV-1 and protein UL49.5 from EHV-1, the genotype of HLA-E of the engineered T cell is 01:01/01:01, and the expression of HLA-A, HLA-B and HLA-E on the cell surface of the engineered T cell is down regulated.

6. A nucleic acid molecule, wherein the nucleic acid molecule is selected from the group consisting of:
(1) a nucleic acid molecules containing a coding sequence of a chimeric antigen receptor and a coding sequence of a functional protein capable of regulating the expression of HLA class I molecules on the cell surface; and
(2) a complementary sequence of the nucleic acid molecule of (1);
preferably, the chimeric antigen receptor and the functional protein is according to claims 3-5.

7. A nucleic acid construct, wherein the nucleic acid construct contains the nucleic acid molecule according to claim 4.

8. The nucleic acid construct according to claim 7, wherein,
the nucleic acid construct comprises an expression frame of the chimeric antigen receptor and an expression frame of the functional protein; or the nucleic acid construct is an expression frame, wherein the coding sequence of the chimeric antigen receptor and the coding sequence of the functional protein are in the expression frame; or
the nucleic acid construct is a cloning vector or an expression vector.

9. A lentivirus containing the nucleic acid construct according to claim 7 or 8.

10. A host cell containing the nucleic acid molecule according to claim 6, or the nucleic acid construct according to claim 7 or 8, or the lentivirus according to claim 9.

11. A pharmaceutical composition, wherein the pharmaceutical composition contains the engineered T cells according to any one of claims 1-5.

12. Use of the functional protein or its coding sequence according to claims 4-5 in the preparation of an engineering modified T cell of which the expression of HLA class I molecules on the cell surface are regulated, or in the preparation of an engineering modified T cell for cancer treatment, wherein the expression of HLA-A and HLA-B and the expression of HLA-E are differentially regulated on the cell surface of the engineering modified T cell, or the expression of HLA-A, HLA-B and HLA-E on the cell surface of the engineered T cell are down regulated.

13. The use according to claim 12, wherein,
the functional protein is selected from protein US 11 from HCMV and protein k5 from KSHV, and the expression of HLA-A and HLA-B and the expression of HLA-E are differentially regulated on the cell surface of the engineered T cell, or
the functional protein is selected from protein US6 from HCMV, protein UL49.5 from BHV-1 and protein UL49.5 from EHV-1, and the genotype of HLA-E of the engineered T cell is 01:03/01:03, or
the functional protein is selected from protein US6 from HCMV, protein UL49.5 from BHV-1 and protein UL49.5 from EHV-1, and the genotype of HLA-E of the engineered T cell is 01:01/01:01.
